(19) Europäisches Patentamt
European Patent Office
Office européen des brevets

(11) EP 1 530 461 B1

(12) EUROPÄISCHE PATENTSCHRIFT

(45) Veröffentlichungstag und Bekanntmachung des Hinweises auf die Patenterteilung:
**03.10.2007 Patentblatt 2007/40**

(21) Anmeldenummer: **04725614.4**

(22) Anmeldetag: **03.04.2004**

(51) Int Cl.:
***A61K 9/70*** *(2006.01)* ***A61K 31/403*** *(2006.01)*
***C07C 219/26*** *(2006.01)*

(86) Internationale Anmeldenummer:
**PCT/EP2004/003574**

(87) Internationale Veröffentlichungsnummer:
**WO 2004/089346 (21.10.2004 Gazette 2004/43)**

# (54) TRANSDERMALE VERABREICHUNG VON (R)-3,3-DIPHENYLPROPYLAMIN-MONOESTERN

TRANSDERMAL ADMINISTRATION OF (R)-3,3-DIPHENYLPROPYLAMINE MONOESTERS

ADMINISTRATION TRANSDERMALE DE MONOESTERS DE (R)-3,3-DIPHENYLPROPYLEAMINE

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR LT LV MK**

(30) Priorität: **08.04.2003 DE 10315878**

(43) Veröffentlichungstag der Anmeldung:
**18.05.2005 Patentblatt 2005/20**

(73) Patentinhaber: **SCHWARZ PHARMA AG**
**40789 Monheim (DE)**

(72) Erfinder:
- **BREITENBACH, Armin**
**51371 Leverkusen (DE)**
- **MEESE, Claus**
**40789 Monheim (DE)**
- **WOLFF, Hans-Michael**
**40789 Monheim (DE)**
- **DREWS, Roland**
**40789 Monheim (DE)**

(56) Entgegenhaltungen:
**EP-A- 0 957 073**
**WO-A-01/35957**
**US-A1- 2002 147 236**
**US-A1- 2003 157 156**
**US-B1- 6 555 129**
**US-B1- 6 638 528**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Arzneimittel zur transdermalen Verabreichung von (R)-3,3-Diphenylpropylamin-Monoestern sowie deren Verwendung zur Herstellung eines Arzneimittels zur transdermalen Verabreichung.

In den vergangenen 50 Jahren ist der Anteil der Senioren innerhalb der Gesamtbevölkerung erheblich gestiegen. In dieser Gruppe gehören Blasenfunktionsstörungen zu den häufigsten Alterskrankheiten. Daher kommt der Entwicklung einer möglichst effektiven und schonenden Therapie von Blasenerkrankungen eine immer größere und besondere Bedeutung zu.

Bei der Dranginkontinenz liegt die Störung in einer Fehlfunktion des Blasenmuskels. Die Ursache ist dabei häufig eine Stimulation bzw. Hyperaktivität der muskarinergen Rezeptoren. Aus diesem Grund werden zur Therapie der hyperaktiven Blase und der damit verbundenen Symptome wie erhöhter Harndrang, Inkontinenz, Pollakisurie oder Nykturie bevorzugt die antimuskarinergen Wirkstoffe Tolterodin und Oxybutynin eingesetzt.

Oxybutynin ist ein effektiver antimuskarinerger Wirkstoff, der jedoch beträchtliche Nebenwirkungen hat. Insbesondere wird von vielen Patienten die ausgeprägte Mundtrockenheit als äußerst unangenehm empfunden.

Tolterodin scheint gegenüber Oxybutynin den Vorteil einer niedrigeren muskarinergen Nebenwirkungsrate aufzuweisen. Tolterodin wird im Organismus vorwiegend über das Cytochrom P 450-lsoenzym 2D6 zum aktiven Hauptmetaboliten 2-[3-(1,1-Düsopropylamino)-1-phenylpropyl]-4-(hydroxymethyl)phenol sowie - langsam - durch das Cytochrom P 450 lsoenzym 3A4 zum inaktiven Metaboliten dealkyliert.

Da Tolterodin ausschließlich über P450-lsoenzyme metabolisiert wird, besteht die potentielle Gefahr der Interaktionen mit dem Abbau anderer Wirkstoffe, z.B. mit Warfarin (Colucci, Annals of Pharmacotherapy 33, 1999, 1173), Antimykotika wie Ketoconazol (Brynne, Br J Clin Pharmacol 48, 1999, 564), Makrolidantibiotika, oder Proteasehemmern.

Diese Gefahr besteht insbesondere bei sogenannten Langsam-Metabolisierern, die einen Mangel an 2D6 haben, Tolterodin ausschließlich über 3A4 metabolisieren und eine deutlich erhöhte Tolterodin-Konzentration im Plasma aufweisen.

Die WO 99/58 478 beschreibt neue Derivate von 3,3-Diphenylpropylaminen als muskarinerge Wirkstoffe. Die offenbarten 3,3-Diphenylpropylamin-Derivate sind Prodrugs von 2-[3-(1,1-Diisopropylamino)-1-phenylpropyl]-4-(hydroxymethyl)phenol und werden beim Durchtritt durch biologische Membranen sowie im Plasma durch Esterasen hydrolysiert. Damit entfällt der 2D6-abhängige Abbaumechanismus.

Solche 3,3-Diphenylpropylamin-Derivate, z.B. 2-[3-(1,1-Diisopropylamino)-1-phenylpropyl]-4-(hydroxymethyl) phenyl isobutyrat (INN: Fesoterodin), neigen daher im Gegensatz zu Tolterodin auch bei Langsam-Metabolisierern nicht zur Akkumulation, interferieren nicht mit P450 Induktoren/Inhibitoren und besitzen im Hinblick auf potentielle Wirkstoffinteraktionen und Wirkstoffakkumulation ein vorteilhaftes Sicherheitsprofil.

Es bestand daher der Bedarf, dem Patientenkollektiv die Vorteile der in WO 99/58478 beschriebenen 3,3-Diphenylpropylamin-Derivate, insbesondere die Vorteile des Fesoterodines, zur Verfügung zu stellen. Gerade der Metabolisierungsweg von Tolterodin und die Nachteile von Oxybutynin (Mundtrockenheit) verdeutlichen den medizinischen Bedarf für ein Arzneimittel, das die Nachteile der beiden vorgenannten Substanzen nicht aufweist.

3,3-Diphenylpropylaminmonoester können als stabile kristalline Salze vorliegen. Ein bevorzugtes Beispiel hierfür ist das Fesoterodin-Hydrogenfumarat. Derartige Salze sind in der WO 01/35957 beschrieben und eignen sich insbesondere zur oralen oder parenteralen Therapie der hyperaktiven Blase.

EP 957 073 offenbart neue 3,3-diphenylpropylamine die unter anderem auch für die Behandlung von Inkontinenz geeignet sind. In einer allgemeinen Liste von möglichen Zubereitungsformen sind auch solche zur transdermalen Verabreichung genannt.

Obwohl die orale Gabe dieser Verbindungen für die meisten Patienten eine geeignete Darreichungsform darstellt, besteht ein Bedarf an einer alternativen Form der Verabreichung. Dieser Bedarf resultiert nicht zuletzt auf dem hohen Alters der von Störungen der Blasenmotilität betroffenen Patienten, bei denen eine Reihe von Gründen gegen die orale Verabreichung von Arzneimitteln sprechen können.

So besteht in diesem Patientenkollektiv häufig eine Multimorbilität, wobei die Patienten in der Regel mehrere verschiedene Medikamente einnehmen. Um Wechselwirkungen mit der Resorption anderer Arzneimittel zu verhindern und/oder den Magendarmtrakt sowie die Leber nicht mit zusätzlichen Arzneimitteln zu belasten, ist eine Umgehung der Darmpassage und der ersten Leberpassage und somit eine nicht-orale Darreichungsform häufig wünschenswert.

Zudem haben zahlreiche ältere Patienten Probleme mit dem Schlucken fester Arzneiformen, während andere betagte Patienten eine gestörte gastrointestinale Absorption, z.B. auf Grund akuter oder chronischer Magen-Darmerkrankungen oder der Einnahme von Antünfektiva, aufweisen.

Schließlich kann mit einer Darreichungsform, die den First-Pass Effekt der ersten Leberpassage vermeidet und die zudem einen Retardierungseffekt aufweist, eine konstantere Plasmakonzentration des Wirkstoffs erreicht werden, was im allgemeinen zu einer Absenkung der Gefahr unerwünschter Nebenwirkungen, insbesondere Mundtrockenheit, bei gleichzeitig unveränderter oder sogar verbesserter klinischer Effektivität führt.

Die transdermale Verabreichung eines 3,3-Diphenylpropylamin-Monoesters und insbesondere von Fesoterodin

ist eine attraktive Option, da sie beispielsweise die mit der oralen Gabe verbundenen Konzentrationsspitzen im Plasma und die damit verbundene Gefahr von muskarinergen Nebenwirkungen, insbesondere Mundtrockenheit, vermeidet.

Gelingt es, über einen längeren Zeitraum eine möglichst gleichmäßige Dosis des Wirkstoffs in den Kreislauf zu transportieren, könnte die Gesamt-Tagesdosis und damit die Effektivität des Wirkstoffs erhöht und gleichzeitig das Auftreten unerwünschter Nebenwirkungen gesenkt werden.

Es war daher ein Ziel der vorliegenden Erfindung, eine Vorrichtung bzw. ein Arzneimittel zur transdermalen Verabreichung von Fesoterodin zur Verfügung zu stellen, das die folgenden Bedingungen erfüllt:

1. Die Vorrichtung sollte eine therapeutisch effektive Tagesdosis eines 3,3-Diphenylpropylamin-Monoesters transdermal verabreichen können.
2. Nach einmaligem Auftrag des Arzneimittels sollte der Wirkstoff über einen längeren Zeitraum, d.h. mindestens über 24 Stunden, bevorzugt über 48 oder 72 Stunden in therapeutisch effektiver Menge durch die Haut abgegeben werden.
3. Der Wirkstoff sollte durch die Haut in einer möglichst konstanten Rate aufgenommen werden, so dass über die vorgesehene Applikationsdauer ein annähernd konstanter Plasmaspiegel aufrecht erhalten wird.
4. Die Hautoberfläche, die mit der Vorrichtung (z.B. dem Pflaster) in Kontakt steht, sollte bevorzugt maximal 50 $cm^2$ groß sein.
5. Auf Hautpenetrationsenhancer sollte möglichst verzichtet werden.
6. Die Vorrichtung sollte möglichst einfach aufgebaut und kostengünstig herstellbar sein.

Die Eignung eines transdermalen Arzneimittels zur kontrollierten, bevorzugt mehrtägigen Wirkstoffgabe wird nun aber von einer Velzahl von Parametern und Erfordernissen beeinflusst, z.B.

- Art der transdermalen Zubereitung (Salbe, Gel, Pflaster, Spray)
- Kontrolle der Wirkstofffreisetzung (passive Diffusion, lontophorese, Ultraschall, Elektroporation)
- der Wirkstoffkonzentration, Beladung und Sättigung der Darreichungsform,
- der Hautdurchgängigkeit für den Wirkstoff unter okklusiven Bedingungen, z.B. nach Auftrag eines Pflasters,
- der Art des Retardierungsprinzips, das eingesetzt wird, um einen kontinuierlichen steady-state Flux über ein oder mehrere Tage zu gewährleisten,
- dem Herstellverfahren der Darreichungsform,
- der erforderlichen Tagesdosis des Wirkstoffs,
- der Verwendung des Wirkstoffs in optimaler Form (Base, Salz, Aggregatzustand, optische Konfiguration).

Ein Arzneimittel zur kontrollierten transdermalen Verabreichung ist somit ein hochkomplexes System, in dem eine Vielzahl von Faktoren oft unvorhersehbaren Einfluß auf die Eigenschaften der Wirkstoffformulierung und die Hautpenetration haben.

So ist trotz langjähriger Bemühungen der pharmazeutischen Industrie bis heute noch keine transdermale Verabreichungsform eines muskarinergen Wirkstoffs, wie z.B. Oxybutynin oder Tolterodin, auf dem Markt erhältlich.

Auch die transdermale Verabreichung des Tolterodin- Hauptmetaboliten 2-[3-(1,1-Diisopropylamino)-1-phenylpropyl]-4-(hydroxymethyl)phenol ist auf Grund seiner sehr niedrigen Penetrationsrate durch Humanhaut klinisch nicht ohne weiteres möglich (siehe Tabelle 1).

In der WO 99/58478 ist ein Hinweis enthalten, dass 3,3,-Diphenylpropylamin-Monoester prinzipiell hautdurchgängig sind, wenn eine Substanzlösung einem Test nach Thiemessen (Acta Pharm Technol 34, 1999, 99) unterzogen wird. Allerdings enthält die WO 99/58478 keine Lehre, wie eine Vorrichtung zur transdermalen Gabe ausgestaltet sein muß, um am Patienten einen möglichst konstanten transdermalen Wirkstoffflux von 3,3-Diphenylpropylaminmonoester über einen längeren Zeitraum zu erreichen.

Zudem erwiesen sich transdermale Arzneiformen, die unter Verwendung der aus WO 01/35957 bekannten hochreinen Salze von 3,3-Diphenylpropylamin-Monoestern hergestellt wurden, in aufwendigen Meßreihen wegen zu niedriger Fluxraten als therapeutisch ungeeignet (Tabelle 1).

Überraschenderweise wurde nun aber gefunden, dass 2-[3-(1,1-Diisopropylamino)-1-phenylpropyl]-4-(hydroxymethyl)phenyl isobutyrat (INN:Fesoterodin) in einer zur therapeutischen Behandlung der überaktiven Blase ausreichende Menge von 0,5-20 mg während eines Zeitraums von mindestens 24 Stunden in konstanter Fluxrate durch Humanhaut befördert werden kann, wenn Fesoterodin in ausreichender Menge als freie Base in eine Polymerschicht, vorzugsweise eine selbstklebende Polymerschicht (Klebermatrix), einer transdermalen Vorrichtung eingebracht wurde.

Mit solch einer einfach aufgebauten flächenförmigen Vorrichtung mit einer Oberfläche von maximal etwa 50 $cm^2$ ist es überraschenderweise möglich, das klinisch relevante Dosierüngsspektrum von Fesoterodin transdermal zur Verfügung zu stellen.

Ein Gegenstand der Erfindung ist daher eine Vorrichtung zur transdermalen Verabreichung von Fesoterodin,

dadurch charakterisiert, dass Fesoterodin in einer Polymerschicht, vorzugsweise in einer selbstklebenden Polymerschicht (Klebermatrix), gelöst oder dispergiert vorliegt und in einer Fluxrate von 0,5-20 mg pro Tag durch Humanhaut freigesetzt wird.

Vorteilhafterweise wird der Wirkstoff in die Polymerschicht, z.B. in die Klebermatrix, in Form der freien Base eingebracht.

Unter dem Ausdruck "freie Base" wird im Sinne der Erfindung verstanden, dass weniger als 20 Gew%, bevorzugt weniger als 10 %, 5 % oder 3%, besonders bevorzugt weniger als 1% von Fesoterodin in Salzform vorliegt.

Werden die aus WO 01/35957 bekannten hochreinen Salze von 3-3-Diphenylpropylamin-Derivaten, z.B. das Fumaratsalz von Fesoterodin, in die Polymerschicht eingebracht, führt dies nur zu therapeutisch unzureichenden transdermalen Fluxraten. Auch der Zusatz geladener Moleküle, wie z.B. Silicate oder Chitosan, oder von Hautpenetrationsverstärken, wie Ölsäure oder Polyglycolmonolaurat zu den Wirkstoffsalz-haltigen Matrices führt nicht zu zufriedenstellenden Fluxraten (Tabelle 1).

Auch eine in-situ Freisetzung der Base aus dem korrespondierenden Salz durch die Zugabe von Calciumsilikat während der Herstellung der Klebermatrix, wie in WO 94/07486 beschrieben, führt in der Regel nicht zu den gewünschten Fluxraten durch Humanhaut (Tabelle 1). Die in-situ Umsetzung zur freien Base verläuft im allgemeinen nicht vollständig, so daß ein zu hoher Anteil des Wirkstoffs in protonierter Form in der Matrix vorliegt.

Bei der Herstellung der erfindungsgemäßen Vorrichtungen sollte Fesoterodin daher der Polymermatrixmasse bevorzugt bereits in Form der freien Base zugesetzt werden. In diesem Fall führten alle getesteten Matrices zu therapeutisch relevanten Fluxraten (Tabelle 1)

Tabelle 1:

| Lot-No | Haftkleber | Verfahren | Wirkstoffbeladung (Gew% Fesoterodin) | Matrixgewicht ($g/m^2$) | Flux $\mu g/cm^2$/Tag (im steady state; nach 24 h) Mäusehaut Humanhaut | |
|---|---|---|---|---|---|---|
| 20111080[1] | Acrylat | Lösungsmittel | 15 | 100 | 705 | n.b. |
| 20302060[1] | Acrylat | Lösungsmittel | 15 | 87 | n.b. | 332,64 |
| 20111085[1] | EVA | Heißschmelz | 15 | 84 | 510 | 323,7 |
| 20111086[1] | Silikon | Heißschmelz | 15 | 63 | 495 | n.b. |
| 20302062[1] | Silikon | Heißschmelz | 15 | 100 | n.b. | 544,89 |
| 20111087[1] | SxS | Heißschmelz | 15 | 89 | 460 | 383,8 |
| 20302063[1] | Silikon + PVAc[6] | Heißschmelz | 15 | 83 | n.b. | 501,09 |
| 20002031[2] | Acrylat | Lösungsmittel | 15 Fumarat | 105 | 27 | n.b. |
| 20104035[2,3] | Acrylat/OL | Lösungsmittel | 15 Fumarat | 110 | 84 | n.b. |
| 20106061[4] | Silicon | Lösungsmittel | 15 Fumarat | 60 | n.b. | 24,2 |
| 20106043[5] | Silikon | Heißschmelz | 15DiOH[5] | 101 | n.b. | 2,3 |

n.b.=nicht bestimmt; [1]=Zur Matrix wurde Fesoterodin als freie Base zugesetzt;
[2]=Vergleichsbeispiel hergestellt durch Verwendung des Fesoterodin-Fumaratsalzes;
[3]=Vergleichsbeispiel hergestellt durch Verwendung des Fesoterodin-Fumaratsalzes mit Ölsäure als Permeationsenhancer; [4]=Vergleichsbeispiel hergestellt durch in-situ Freisetzung der Base aus dem Fumaratsalz in der Klebermatrix; [5]=Vergleichsbeispiel hergestellt durch Verwendung des Dihydroxymetaboliten (2-[3-(1,1-Diisopropylamino)-1-phenylpropyl]-4-(hydroxymethyl)phenol) von Fesoterodin; [6]PVAc = Polyvinylacetat.

Bevorzugt sollte der Salzanteil von Fesoterodin daher möglichst gering sein. Typischerweise sollte der Teil von Fesoterodin, der in Salzform in die Polymermatrix eingebracht wird, weniger als 20 Gew%, bevorzugt weniger als 10 %, 5 % oder 3% und besonders bevorzugt weniger als 1% des Gesamtgewichts des eingesetzten Wirkstoffs betragen.

Erfindungsgemäß liegt Fesoterodin zu über 90%, bevorzugt zu über 95% besonders bevorzugt zu über 99% als optisch hochreines Enantiomer in (R)-Konformation vor. Dies bedeutet, dass weniger als 10%, bevorzugt weniger als 3%, besonders bevorzugt weniger als 1 % der Verbindung in (S)-Konfiguration vorliegt.

In einer bevorzugten Ausführungsform der Erfindung hat die Polymermatrix ein Gewicht von 30-300 $g/m^2$,

enthält 50-95 Gew% eines Polymers, bevorzugt eines selbstklebenden Polymers (Haftklebers), und 5-40 Gew% Fesoterodin (jeweils bezogen auf das Gesamtgewicht der Polymermatrix).

In einer besonders bevorzugten Ausführungsform der Erfindung ist die Vorrichtung dadurch charakterisiert, dass sie

(a) eine Grundfläche von maximal 50 $cm^2$ aufweist,
(b) eine selbstklebende Polymerschicht umfasst, die

(b1) ein Gewicht von 30-300 $g/m^2$ aufweist,
(b2) 50-95 Gew% eines Haftklebers enthält,
(b3) Fesoterodin in einer Konzentration von 5-40 Gew% bezogen auf das Gesamtgewicht der Polymerschicht enthält und besonders bevorzugt,

(c) Fesoterodin über einen Zeitraum von mindestens 24 Stunden mit einer steady-state Fluxrate von mindestens 4 $\mu g/cm^2$/Stunde durch Humanhaut abgibt.

Unter dem Ausdruck "Polymer" werden In dieser Patentanmeldung auch Copolymere subsumiert.

Unter dem Ausdruck "Polymermatrix" wird In der vorliegenden Erfindung eine Schicht, Lage oder Masse verstanden, die ein oder mehrere Polymere umfasst. Ist die Polymermatrix selbstklebend, wird sie auch als "Klebermatrix" bezeichnet.

Unter dem Ausdruck Gesamtgewicht der Polymermatrix" wird in dieser Patentanmeldung das Gewicht der Polymermatrix inklusiv des darin eingebrachten Wirkstoffs und etwaiger Hilfsstoffe verstanden.

Bei der erfindungsgemäßen Vorrichtung handelt es sich um eine üblicherweise flächenförmige transdermale Vorrichtung/Arzneiform vom Matrix-Typ, das heißt, der Wirkstoff liegt in eine Polymerschicht oder -masse eingebettet (gelöst oder dispergiert) vor.

Vorzugsweise enthält die Vorrichtung eine monolithische Klebermatrix, in die der Wirkstoff eingebettet ist.

Ein Beispiel für einen typischen Aufbau einer monolithischen transdermalen Vorrichtung ist in Abbildung 4 wiedergegeben. Die dort beschriebene Vorrichtung besteht aus der wirkstoffhaltigen Klebermatrix (1), einer für die Inhaltsstoffe der Klebermatrix inerten und undurchlässigen Rückschicht, die sich nach dem Auftrag des Pflasters auf der Haut des Patienten auf der der Haut abgewandten Seite des Pflasters befindet (2) sowie einer die Klebermatrix bei Lagerung schützenden, unmittelbar vor dem Gebrauch ablösbaren Schicht (3).

Die Fläche der transdermalen Vorrichtung beträgt erfindungsgemäß maximal 50 $cm^2$, bevorzugt maximal 40 $cm^2$. Besonders bevorzugte Pflastergrößen liegen zwischen 5 und 35 $cm^2$, ganz besonders bevorzugt zwischen 10 und 30 $cm^2$.

Die den Haftkleber und den Wirkstoff enthaltende Polymermatrix, z.B. die Klebermatrix, hat typischerweise eine Dicke von 30-300 $\mu$m, bevorzugt von 40-200 $\mu$m und ein Durchschnittsgewicht von 30-300 $g/m^2$, bevorzugt von 40-200 $g/m^2$.

Der Wirkstoff liegt in der Polymermatrix, vorzugsweise in der Klebermatrix erfindungsgemäß in einer Konzentration von 5-40 Gew%, bevorzugt 7-30 Gew% und besonders bevorzugt 8-20 Gew% bezogen auf das Gesamtgewicht der Klebermatrix vor, wenn die Vorrichtung/das Arzneimittel zum Beispiel zur 2-3 tägigen Applikation vorgesehen ist. Soll ein Arzneimittel zur 7-tägigen Verabreichung von Fesoterodin hergestellt werden, werden Wirkstoffkonzentrationen über 15 Gew%, bevorzugt 20-40 Gew% eingesetzt.

Die Wirkstoffbeladung der Polymerschicht, z.B. der Klebermatrix, kann daher zwischen 0,15 und 12 $mg/cm^2$ liegen. Bevorzugte Beladungsstärken sind Beladungen zwischen 0,25 und 7,5 $mg/cm^2$, besonders bevorzugt zwischen 0,32 und 4 $mg/cm^2$. Bei Vorrichtungen zur 7-tägigen Applikation liegt die Beladung bevorzugt bei mindestens 2 $mg/cm^2$.

In einer Ausführungsform der Erfindung liegt die freie Base von Fesoterodin dabei in einer Konzentration vor, die zur Übersättigung der jeweiligen Polymermatrix mit Wirkstoff führt. Dies kann zur Bildung von sogenannten Mikroreservoiren führen, die tropfenförmig in der Matrix, insbesondere in einer hydrophoben Matrix, z.B. vom Silikontyp, vorliegen.

In diesem Fall wird bevorzugt, dass die Wirkstoff-haltigen Mikroreservoire in der Matrix eine möglichst gleichmäßige mittlere Größenverteilung von maximal 50% der Schichtdicke der Matrix aufweisen. Dies kann durch eine intensive Homogenisierung der Matrix bei der Herstellung gewährleistet werden, so daß besonders bevorzugt eine mittlere Tröpfchengröße von bis zu 30 $\mu$m resultiert.

In einer anderen Ausführungsform der Erfindung liegt der Wirkstoff in einer Konzentration vor, in der er vollständig in der Matrix, z.B. in einer Acrylatmatrix, gelöst ist.

Die zur Herstellung der erfindungsgemäßen Vorrichtungen verwendeten freien Basen von Fesoterodin lassen sich prinzipiell gewinnen, wie in WO 99/58478 offenbart. Hierzu wird (R)- 2-(3-Diisopropylamino-1-phenylpropyl)-4-hydroxymethylphenol unter basischen Bedingungen mit einem geeigneten Säurechlorid, z.B. Isobuttersäurechlorid,

umgesetzt.

Diese Reaktion führt jedoch nur zu ca 90% bis maximal ca. 94% des gewünschten Hauptproduktes (B). Das Produkt enthält regelmäßig 6-10% Verunreinigungen der Ausgangssubstanz (A) sowie ungewünschte Reaktionsprodukte in Form des entsprechenden Diesters (C), bzw. durch Bildung des Monoesters (D) der 4-Hydroxygruppe (siehe Abbildung 1) sowie durch Polymerisierung.

Für pharmazeutische Präparate wird aber in der Regel eine Reinheit von über 97 Gew% bevorzugt.

Es wurde gefunden, dass eine freie Base von Fesoterodin in einer Reinheit von regelmäßig über 97 Gew%, bevorzugt über 98 Gew%, besonders bevorzugt über 98,5 Gew% und ganz besonders bevorzugt über 99 Gew% in hoher Ausbeute von in der Regel über 90 Mol % gewonnen werden kann, wenn die freie Base hergestellt wird, in dem sie mit einem geeigneten Reagenz aus einem hochreinen kristallinen Salz freigesetzt wird.

Unter dem Ausdruck "hochrein" wird in dieser Anmeldung ein Reinheitsgrad von mindestens 97 Gew%, bevorzugt über 98 Gew%, besonders bevorzugt über 98,5 Gew% und ganz besonders bevorzugt über 99 Gew% verstanden. Der Reinheitsgrad wird dabei bestimmt wie im Methodenteil beschrieben.

Die erfindungsgemäßen hochreinen Basen von Fesoterodin können hergestellt werden, indem sie aus hochreinen kristallinen Salzen der allgemeinen Formel II freigesetzt werden:

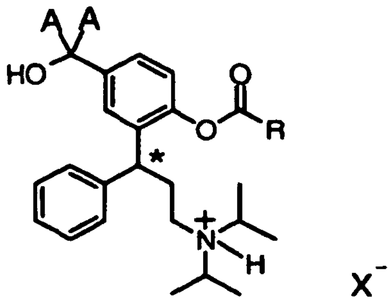

Formel II

wobei A Wasserstoff repräsentiert, R für Isopropyl steht und X- der Säurerest einer physiologisch verträglichen Säure ist, und wobei das mit "*" (Stern) markierte C-Atom in (R)-Konfiguration, in (S)-Konfiguration oder als Mischung davon vorliegen kann.

Als Säurerest X kommt dabei insbesondere das Anion einer der nachfolgend genannten Säuren in Betracht: Salzsäure, Bromwasserstoffsäure, Phosphorsäure, Schwefelsäure, Salpetersäure, Essigsäure, Propionsäure, Palmitinsäure, Stearinsäure, Maleinsäure, Fumarsäure, Oxalsäure, Bernsteinsäure, DL-Äpfelsäure, L-(-)-Äpfelsäure, D-(+)-Äpfelsäure, DL-Weinsäure, L-(+)-Weinsäure, D-(-)-Weinsäure, Citronensäure, L-Asparaginsäure, L-(+)-Ascorbinsäure, D-(+)-Glucuronsäure, 2-Oxopropionsäure (Brenztraubensäure), Furan-2-carbonsäure (Brenzschleimsäure), Benzoesäure, 4-Hydroxybenzoesäure, Salicylsäure, Vanillinsäure, 4-Hydroxyzimtsäure, Gallussäure, Hippursäure (N-Benzoylglycin), Acetursäure (N-Acetylglycin), Phloretinsäure (3-(4-Hydroxyphenyl)-propionsäure), Phthalsäure, Methansulphonsäure oder Orotsäure, wobei die Säureanionen Hydrogenfumarat und Hydrochlorid besonders bevorzugt werden.

Aus dieser hochreinen Verbindung der allgemeinen Formel II werden die korrespondierenden hochreinen freien Basen durch Zugabe entsprechender Reagenzien freigesetzt.

Freisetzungsreagenzien sind z.B. alkalische Verbindungen aus der Gruppe der

- Hydroxide, Carbonate und Alkali-, Erdalkali- oder Ammonium-Hydrocarbonate
- Amine, Polyamine und basischen Polyaminosäuren, die sowohl in Lösung als auch auf Trägern fixiert vorliegen können,
- basischen Ionentauscher,

wobei schwach alkalische Verbindungen mit einem $PK_B$ von 8-11 bevorzugt werden.

Als "Freisetzungsreagenz" besonders bevorzugt wird ein Alkali-, Erdalkali- oder Ammonium-Hydrogencaronat, wobei Natrium-Hydrogencarbonat ganz besonders bevorzugt wird.

In einem bevorzugten Herstellverfahren wird zunächst Salz der Formel II in Wasser aufgenommen und mit einem basischen Freisetzungsreagens, z.B. einem Hydrogencarbonat, versetzt. Dann wird mit einem geeigneten Lösemittel ausgeschüttelt und die organische Phase eingeengt, bis hochreine Base von Fesoterodin als viskoses Öl zurückbleibt. Ein solches Verfahren wird näher in Ausführungsbeispiel C. dargestellt.

Geeignete Lösemittel für solche Verfahren sind insbesondere Dichlormethan, tert-Butylmethylether, Diethylether, Ethylmethylketon sowie Toluol, wobei Dichlormethan bevorzugt wird.

In einem alternativen Herstellverfahren wird das hochreine Salz der Formel II in einem geeigneten Lösemittel

aufgenommen und dann über einen Träger geleitet, der beispielsweise immobilisierten Ionentauscher enthält. Das Eluat enthält dann die hochreine Base von Fesoterodin.

Besonders bevorzugt wird zur Herstellung der hochreinen freien Base von (R)-2-[3-(1,1-Diisopropylamino)-1-phenylpropyl]-4-(hydroxymethyl)phenyl isobutyrat als Ausgangsverbindung der Formel II (R)-2-[3-(1,1-Diisopropylamino)-1-phenylpropyl]-4-(hydroxymethyl)phenyl isobutyrat hydrogenfumarat eingesetzt.

Die Herstellung der hochreinen Salze der Formel II ist aus WO 01/35957 bekannt. Hierzu wird zunächst eine Lösung von 2-[3-(1,1-Diisopropylamino)-1-phenylpropyl]-4-(hydroxymethyl)phenol in basischer Lösung mit einem Säurechlorid, z.B. Isobuttersäurechlorid, umgesetzt. Die resultierende Base mit niedrigem Reinheitsgehalt wird dann unter Erwärmen mit einer Säure, z.B. Fumarsäure, versetzt. Das entstandene Salz der allgemeinen Formel II lässt sich in geeigneten Lösungsmitteln auskristallisieren. Die Kristalle werden erneut gelöst und rekristallisiert. Dieser Prozess kann gegebenenfalls wiederholt werden, bis eine Verbindung der Formel II mit dem gewünschten Reinheitsgrad erhalten wird. Aus diesen Salzen wird die hochreine freie Base von Fesoterodin wie oben beschrieben freigesetzt.

Tabelle 2 zeigt die Aufreinigung von Fesoterodin-Base durch das oben beschriebene Verfahren

| **Verfahrensschritte [a)]** | **Reinheit B oder E (%)** |
|---|---|
| 1. chemische Synthese von B aus A | 94,37 |
| 2. Herstellung des Salzes E aus B (1.) | 92,58 |
| 3. Umkristallsierung des Salzes E aus (2.) | 99,32 |
| 4. Freigesetzte hochreine Base B aus E (3.) | 99,14 |
| a) A, B, C, E: R = i-Pr, s. Abbildung 1 | |

Das beschriebene Verfahren erlaubt erstmals die effiziente Darstellung der freien Base von Fesoterodin in hochreiner Form und damit auch erstmals die Verwendung der hochreinen Basen von Fesoterodin zur Herstellung der erfindungsgemäßen Vorrichtungen zur kontrollierten transdermalen Verabreichung.

Ein bevorzugter Gegenstand der Erfindung ist daher eine Vorrichtung zur transdermalen Verabreichung von Fesoterodin, dadurch charakterisiert, dass die Vorrichtung hergestellt wird, indem Fesoterodin als freie Base mit einem Reinheitsgrad von 97 Gew%, bevorzugt über 98 Gew%, besonders bevorzugt über 98,5 Gew% und ganz besonders bevorzugt über 99 Gew% in eine Polymerschicht, bevorzugt in eine selbstklebenden Polymermatrix (Klebermatrix) eingebracht wird.

Ein besonders bevorzugter Gegenstand der Erfindung ist eine flächenförmige Vorrichtung zur transdermalen Verabreichung von Fesoterodin, dadurch charakterisiert, dass die Vorrichtung umfasst

(a) eine Grundfläche von maximal 50 $cm^2$,
(b) eine selbstklebende Polymermatrix, die

(b1) ein Gewicht von 30-300 $g/m^2$ aufweist,
(b2) 50-95 Gew% eines Haftklebers enthält,

(c) in der selbstklebenden Polymermatrix (b) gelöstes oder dispergiertes Fesoterodin, das

(c1) in einer Konzentration von 5-40 Gew% bezogen auf das Gesamtgewicht der Polymermatrix vorliegt,
(c2) bevorzugt in Form der freien Base mit einem Salzgehalt von weniger als 10 Gew% sowie
(c3) vorzugsweise in einem Reinheitsgrad von über 97 Gew%, bevorzugt über 98 Gew%, besonders bevorzugt über 98,5 Gew% und ganz besonders bevorzugt über 99 Gew% in besagte Polymerschicht eingebracht wird.

Zur Herstellung der Polymerschicht kommen diverse in der Pflastertechnologie bekannte Polymere in Frage, wobei die Verwendung haftklebender Polymere, wie weiter unten näher erläutert, bevorzugt wird.

Im allgemeinen wird der Flux eines bestimmten Wirkstoffs durch Humanhaut wesentlich bestimmt durch die verwendete Matrix, in der der Wirkstoff eingebettet ist. Im vorliegenden Fall führten entgegen der Erwartung alle verwendeten selbstklebenden Matrices in-vitro zu hohen Fluxraten durch Säugerhaut (Abbildung 3, Tabelle 1). Der Grund hierfür sind die außergewöhnlich guten Hautpenetrations-Eigenschaften der freien Basen von Fesoterodin.

Es wird davon ausgegangen, dass im Durchschnitt eine effektive Tagesdosis von 0,5-20 mg, üblicherweise von mindestens 3 mg/Tag, z.B. 3-20 mg/Tag, bevorzugt 3-15 mg/Tag und besonders bevorzugt 4-12 mg/Tag des Wirkstoffs, bzw. des aktiven Metaboliten (R)-2-[3-(1,1-Diisopropylamino)-1-phenylpropyl]-4-(hydroxymethyl)-phenol

durch die Haut eines Patienten befördert werden muss. In Ausnahmefällen kann eine geringere Tagesdosis von 0,5-3 mg/Tag oder eine höhere Dosis von über 20 mg/Tag hinreichend bzw. erforderlich sein.

**Tabelle 3 zeigt, dass TTS, bei denen die hochreine Base von (R)- Fesoterodin in einer Menge von 15 Gew% in geeignete Klebermatrices vom SXS- oder EVA-Typ eingebracht wurde, bei in-vitro Versuchen mit Humanhaut zu Fluxraten führen, die bei entsprechender Auftragsfläche von 5-50 $cm^2$ therapeutisch gewünschte Tagesdosen wie folgt ermöglichen:**

| Fluxrate Fesoterodin durch Humanhaut (mg/Tag) bezogen auf die TTS-Größe | | | | | | |
|---|---|---|---|---|---|---|
| TTS-Grösse / Haftkleber | 5 $cm^2$ | 10 $cm^2$ | 20 $cm^2$ | 30 $cm^2$ | 40 $cm^2$ | 50 $cm^2$ |
| EVA | 1,6 | 3,2 | 6,5 | 9,7 | 13 | 16 |
| SXS | 1,9 | 3,8 | 7,6 | 11,4 | 15,2 | 19 |
| Silikon/Cer + PVAc | 2,5 | 5 | 10 | 15 | 20 | 25 |
| Acrylat (Durotak 87-4287) | 1,7 | 3,3 | 6,6 | 10 | 13,3 | 16,7 |

Überraschenderweise lieferte das erfindungsgemäße, Fesoterodin als freie Base enthaltende Arzneimittel in den getesteten Matrices transdermale steady-state Fluxraten durch Humanhaut von über 300 $\mu g/cm^2$/Tag, d.h. von über 13 $\mu g/cm^2$/h über einen Zeitraum von mindestens 48 Stunden (Abbildung 2).

Dabei übersteigen die erzielbaren in-vitro Fluxraten durch Humanhaut aus den erfindungsgemäßen Polymermatrices diejenigen aus freier Lösung, die in WO 99/58478 angegeben wurden, überraschend deutlich.

Durch die konstant hohen Fluxraten ermöglichen die erfindungsgemäßen Matrices aber überraschenderweise die Herstellung von Vorrichtungen zur kontrollierten transdermalen Verabreichung von (R)- 2-[3-(1,1-Diisopropylamino)-1-phenylpropyl]-4-(hydroxymethyl)phenyl isobutyrat (Fesoterodin freie Base) über den gesamten therapeutisch relevanten Dosierungsbereich von 0,5-20 mg/Tag und in konstanter Fluxrate über mindestens 24 Stunden, ohne eine Gesamtoberfläche von 50 $cm^2$ signifikant zu überschreiten.

Ein Aspekt der vorliegenden Erfindung ist daher ein Arzneimittel zur transdermalen Verabreichung von (R)-2-[3-(1,1-Diisopropylamino)-1-phenylpropyl]-4-(hydroxymethyl)phenyl isobutyrat (Fesoterodin freie Base) über einen Zeitraum von mindestens 24 Stunden, bevorzugt mindestens 48 Stunden, in einer konstanten Fluxrate von mehr als 125 $\mu g$/h, bevorzugt 125-850 $\mu g$/h, besonders bevorzugt 125-650 $\mu g$/h und ganz besonders bevorzugt 150-500 $\mu g$/h.

Ein anderer Aspekt der Erfindung ist ein Arzneimittel zur konstanten transdermalen Verabreichung von (R)-2-[3-(1,1-Diisopropylamino)-1-phenylpropyl]-4-(hydroxymethyl)phenyl isobutyrat (Fesoterodin freie Base) über einen Zeitraum von mindestens 24 Stunden, bevorzugt mindestens 48 Stunden in einer steady-state Fluxrate von mehr als 4 $\mu g/cm^2$/h, bevorzugt von mehr als 6 $\mu g/cm^2$/h, besonders bevorzugt von mehr als 8 $\mu g/cm^2$/h, 10 $\mu g/cm^2$/h oder mehr als 12 $\mu g/cm^2$/h, wobei die Fluxraten in einem in-vitro Humanhautmodell nach Tanojo bestimmt werden, wie in Ausführungsbeispiel 3.2 angegeben.

Das bei der vorliegenden Erfindung verwendete Humanhaut-Modell nach Tanojo hat sich als ausgezeichnetes Modell erwiesen, in dem die gemessenen in-vitro-Fluxraten mit den in-vivo Fluxraten und Plasmaspiegeln korrelierten, die in mehreren klinischen Studien mit verschiedenen aminohaltigen Wirkstoffen gemessen wurden.

Die mit den erfindungsgemäßen Vorrichtungen gemessenen täglichen Fluxraten entsprechen der Menge, die sich in klinischen Studien bei oraler Verabreichung als bei den meisten Patienten optimale effektive Dosis von (R)-2-[3-(1,1-Diisopropylamino)-1-phenylpropyl]-4-(hydroxymethyl)phenyl isobutyrat (Fesoterodin) herausgestellt hat (ca. 4-12 mg pro Tag) und die zu therapeutischen Plasmaspiegeln von 1-15 ng/ml, bevorzugt von 2-12 ng/ml, besonders bevorzugt von 3-10 ng/ml des Metaboliten (R)-2-[3-(1,1-Diisopropylamino)-1-phenylpropyl]-4-(hydroxymethyl)phenol führt.

Ein anderer Aspekt der Erfindung ist daher ein Arzneimittel zur transdermalen Verabreichung von (R)- 2-[3-(1,1-Diisopropylamino)-1-phenylpropyl]-4-(hydroxymethyl)phenyl isobutyrat (Fesoterodin freie Base) in einer Fluxrate, die zu Herstellung eines konstanten Plasmaspiegels von 1-15 ng/ml, bevorzugt von 2-12 ng/ml, besonders bevorzugt 3-10 ng/ml des Metaboliten (R)-2-[3-(1,1-Diisopropylamino)-1-phenylpropyl]-4-(hydroxymethyl)phenol über einen Zeitraum von mindestens 24, bevorzugt mindestens 36 Stunden führt.

Unter dem Ausdruck "steady-state" wird in der vorliegenden Patentanmeldung ein Fließgleichgewicht verstanden, das sich nach einer initialen lag-Phase nach dem erstmaligen Auftragen der erfindungsgemäßen Vorrichtung einstellt.

Unter "steady-state Fluxrate" wird eine Fluxrate verstanden, die sich nach der initialen lag Phase einstellt.

Unter dem Ausdruck "konstante Fluxrate" wird in dieser Patentanmeldung eine steady-state Fluxrate verstanden, bei Fesoterodin in einer mittleren Fluxrate durch Humanhaut transportiert wird, die eine intraindividuelle Variabilität CV über die Zeit von maximal 30 %, bevorzugt maximal 20 % oder sogar maximal 10 % aufweist, wobei CV nach der Gleichung CV = (sd : x ) x 100 % bestimmt wird (siehe Berechnung Cawello (ED) in "Parameters for Compartment-free Pharmacokinetics", Shaker Verlag, Aachen, 1999, Seite 112). Eine Tagesdosis wird dabei in einer mittleren Fluxrate von Tagesdosis:24 (mg/Stunde) mit einer CV von 30 % verabreicht. Für den Fachmann ist klar, dass sich eine konstante Fluxrate erst nach einer initialen Anflutungsphase ("lag-Phase") nach erstmaligem Auftrag der Vorrichtung einstellt. Die lag-Phase wird daher bei der Berechnung der konstanten Fluxrate nicht berücksichtigt.

Unter dem Ausdruck "Fluxrate durch Humanhaut" wird in dieser Patentanmeldung, sofern nicht ausdrücklich anders angegeben, eine Fluxrate verstanden, die im in vitro-Humanhautmodell nach Tanojo, wie in Ausführungsbeispiel 3.2 beschrieben, gemessen wurde.

Unter dem Ausdruck "konstanter Plasmaspiegel" wird in dieser Patentanmeldung verstanden, dass der Patient nach einer initialen Anflutungsphase nach dem ersten Auftragen der erfindungsgemäßen Arzneimittel über wenigstens 80%, bevorzugt wenigstens 85% und besonders bevorzugt wenigstens 90% der Zeit der Administration der erfindungsgemäßen Arzneimittel einen definierten Plasmaspiegel des aktiven Metaboliten aufweist.

Die erfindungsgemäßen Vorrichtungen enthalten in der Polymerschicht im allgemeinen 50-95 Gew%, bevorzugt 70-90 Gew% eines Polymers, bevorzugt eines haftklebenden Polymers ("Haftkleber").

Als Basis für eine selbstklebende Polymerschicht kommen dabei grundsätzlich die in der Pflastertechnologie bekannten Haftkleber in Frage, wie beispielsweise Silikonkleber, Ethylvinylacetat (EVA-)-Kleber, Styrol-Block-Copolymer (SXS)-Kleber, Acrylatkleber, Polyurethankleber, Vinylacetatkleber sowie adhesive Gummen, z.B. Polyisobutylene, Polybutadiene, Neoprene oder Polyisoprene sowie geeignete Mischungen dieser Kleber.

Ein Gegenstand der Erfindung ist daher eine Vorrichtung zur transdermalen Verabreichung von Fesoterodin, die dadurch charakterisiert ist, dass Fesoterodin vorzugsweise in Form der freien Base in eine selbstklebende Polymerschicht eingebracht ist, wobei die selbstklebende Polymerschicht mindestens einen Haftkleber aus der Gruppe der Silikonkleber, Ethylvinylacetat (EVA-)-Kleber, Styrol-Block-Copolymer (SXS)-Kleber, Acrylatkleber, Polyurethankleber, Vinylacetatkleber, der adhesiven Gummen, z.B. Polyisobutylene, Polybutadiene, Neoprene oder Polyisoprene oder geeignete Mischungen dieser Kleber umfasst.

Die Herstellung der wirkstoffhaltigen Polymerschichten kann grundsätzlich im Lösemittelverfahren oder im Heißschmelzverfahren erfolgen.

Im Lösemittelverfahren werden Wirkstoff und Polymer sowie etwaige weitere Hilfsmittel jeweils in Lösemitteln gelöst und dann miteinander vermischt. Die Mischung wird dann auf Folie ausgestrichen und die Lösemittel unter Erwärmung abgedampft.

Im Heißschmelzverfahren werden die Polymere sowie etwaige Hilfsstoffe ohne Verwendung von Lösemitteln thermisch aufgeschmolzen, gemischt und die Schmelze auf Folie ausgestrichen. Der Wirkstoff wird in der Regel direkt in die flüssige Schmelze eingebracht. Für das Heißschmelzverfahren eignen sich insbesondere solche Polymere, die bei Verarbeitungstemperaturen von bis zu 200 °C ausreichend flüssig sind, d.h. bevorzugt eine dynamische Viskosität von unter 100 Pa.s aufweisen. Die dynamische Viskosität kann dabei bestimmt werden, wie in US 5,328,696 beschrieben.

Die Verbindungen der allgemeinen Formel, insbesondere Fesoterodin, erwiesen sich bei den Bearbeitungstemperaturen bis 200°C im Heißschmelzverfahren als überraschend stabil.

Als Haftkleber besonders geeignet sind die bekannten Polymere vom Acrylat-Typ, vom SxS-Typ, vom EVA-Typ sowie vom Silikon-Typ, insbesondere heißschmelzfähige Silikon-Weichmacher-Mischungen. Diese Kleber weisen eine ausreichende Haftfähigkeit auf Humanhaut auf und liefern bezüglich der hochreinen Basen von Fesoterodin, ausgezeichnete Fluxraten. Diese Kleber sind gut hautverträglich und für pharmazeutische Zwecke geeignet. Die Eigenschaften und die Herstellung dieser bevorzugten Haftkleber werden im folgenden beispielhaft näher erläutert:

<u>Silikonkleber:</u>

Bevorzugte Silikonhaftkleber sind amin-resistente, druck-sensitive Polyorganosiloxankleber.

Silikonhaftkleber stellen in den meisten Fällen Polydimethylsiloxane dar, allerdings können prinzipiell statt Methylgruppen auch andere organische Reste, wie z.B. Ethyl oder Phenylgruppen vorhanden sein. Aminresistente

Silikonhaftkleber zeichnen sich im allgemeinen dadurch aus, dass sie keine oder nur wenige freie Silanolfunktionen enthalten, da die Si-OH-Gruppen alkyliert wurden. Solche Kleber sind in der EP 180 377 beschrieben. Besonders bevorzugte Kleber sind Kondensate oder Mischungen von Silikonharzen und Polyorganosiloxanen, wie beispielsweise in US RE 35 474 beschrieben.

Geeignete Kleber werden beispielsweise von Dow Corning als sogenannte Bio-PSA Kleber vertrieben. Besonders geeignet sind dabei Mischungen der Haftkleber Bio PSA Q7-4301 und Q7-4201 insbesondere im Verhältnis 40:60 bis 60:40.

Pflaster-Matrices auf Basis von Silikonklebern werden überwiegend in lösungsmittelbasierten Verfahren verarbeitet. Hierzu wird im ersten Schritt eine Lösung aus Haftklebern und Wirkstoff in einem organischen Lösungsmittel oder einem Lösungsmittelgemisch hergestellt. Im zweiten Schritt wird die Lösung ausgestrichen und laminiert und dann das Lösungsmittel entfernt. Ein solches Verfahren ist beispielsweise beschrieben in WO 99/49852.

Ein alternatives Verfahren, das auf die Verwendung von organischen Lösungsmitteln verzichtet, ist das Heißschmelzverfahren. Bei diesem Verfahren wird das Polymer bzw. der Haftkleber bei Temperaturen zwischen 70 und 200°C, bevorzugt zwischen 90 und 160°C und besonders bevorzugt zwischen 100 und 150 °C geschmolzen und der Wirkstoff in die homogenisierte Matrixschmelze gebracht. Nach kurzer Homogenisierung wird die wirkstoffhaltige Klebermatrix wieder abgekühlt, so daß der Wirkstoff im allgemeinen für weniger als 5 Minuten, falls gewünscht sogar weniger als 4, 3, 2 oder sogar weniger als 1 Minute thermischer Belastung ausgesetzt wird. Danach liegt der Wirkstoff in der erstarrten Polymerschmelze vor. Während des Prozesses ist der Wirkstoff von kritischen Umwelteinflüssen (Licht, Sauerstoff) weitgehend abgeschirmt.

Dieses Verfahren hat gegenüber dem lösungsmittelbasierten Verfahren den Vorteil, dass die hochreinen Basen von keinen Lösemitteleinflüssen ausgesetzt werden, sondern sofort in die heiße Schmelze gegeben werden können, wo sie nach kurzer Homogenisierung in der erkaltenden Polymermatrix stabilisiert werden. Das Heißschmelzverfahren wird bevorzugt in einem Extruder, z.B. in einem Doppelschneckenextruder durchgeführt, wie in WO 99/48493 beschrieben.

Silikonkleber sind bei den oben genannten Verarbeitungstemperaturen zu viskos, d.h. sie haben eine dynamische Viskosität von über 150 Pa.s. In der Patentliteratur wurden verschiedene Verfahren beschrieben, die Viskosität von Silikonklebern durch Beimischung von geeigneten Zusätzen (Weichmachern) heißschmelzfähig zu machen. Beispiele für solche Weichmacher für Silikone sind Glycerolmonolaurat oder Laurylactetat, wie in EP 835 136 beschrieben, Wachse der Formel R-C(O)-OR' wie in EP 360 467 beschrieben, Alkylmethylsiloxanwachse wie in EP 524 775 beschrieben, siloxierte Polyetherwachse, wie in EP 663 431 beschrieben oder organische Wachse, wie in US RE 36 754 beschrieben.

Die Weichmacher werden dem Silikonkleber im allgemeinen in einer Menge von 1-30 Gew% bezogen auf das Gesamtgemisch der heißschmelzfähigen Klebermischung zugesetzt. Bevorzugte Weichmacher sind organische Wachse, wie in US RE 36 754 beschrieben, z.B. Ozokerit, Ceresin, Paraffin, Candelila, Carnauba, Bienenwachs oder Mischungen dieser Wachse, wobei Ozokerit und Ceresin ganz besonders bevorzugt werden.

Vorgefertigte heißschmelzfähige Silikonhaftkleber, insbesondere Mischungen aus Silikonhaftklebern mit Ceresin oder Ozokerit, können bei Dow Corning, Michigan, bezogen werden. Durch den Zusatz von 10 Gew% Ceresin zu einem Silikon-Haftkleber vom Typ Q7-4301 gelingt es beispielsweise, die dynamische Viskosität des resultierenden Haftklebergemischs bei einer Verarbeitungstemperatur von 150°C von über 200 Pa.s auf unter 50 Pa.s zu senken. Eine solche silikonbasierte Haftklebermischung kann in einem Temperaturbereich von 70°C bis 200°C und insbesondere im Bereich zwischen 100°C und 150°C sehr gut im Heißschmelzverfahren verarbeitet werden.

Überraschenderweise wurde festgestellt, daß heißschmelzfähige Silikonhaftkleber ausgezeichnet zur transdermalen Verabreichung von Fesoterodin geeignet sind.

Ein Gegenstand der Erfindung ist daher eine Vorrichtung zur transdermalen Verabreichung von Fesoterodin, dadurch charakterisiert, dass Fesoterodin in einer selbstklebenden Polymerschicht (Klebermatrix) vorliegt, wobei die Klebermatrix ein aminoresistentes Silikon umfasst.

Vorzugsweise wird Fesoterodin in Form der freien Base, besonders bevorzugt in Form der hochreinen freien Base in die Silikonmatrix eingebracht.

In einer besonders bevorzugten Ausführungsform der Erfindung basiert die Klebermatrix auf einer heißschmelzfähigen Mischung aus einem silikonbasierten Haftkleber und mindestens einem Weichmacher, insbesondere einem organischen Wachs, z.B. Ozokerit.

Ein weiterer Aspekt der Erfindung ist ein Arzneimittel zur transdermalen Verabreichung von Fesoterodin, umfassend eine Klebermatrix, die umfasst:

(a) 50-99 Gew% einer Haftklebermischung bestehend aus

(i) 70-99 Gew% eines aminoresistenten Silikonklebers,
(ii) 1-30 Gew%, bevorzugt 3-15 Gew% eines geeigneten Weichmachers, bevorzugt eines organischen Wachses,

welches besonders bevorzugt ausgewählt wird aus der Gruppe Ozokerit, Ceresin, Paraffin, Candelilla, Carnauba, Bienenwachs oder Mischungen dieser Wachse, wobei Ozokerit und Ceresin besonders bevorzugt werden,

(b) 1-40 Gew% Fesoterodin, das besonders bevorzugt in Form der freien Base und ganz besonders bevorzugt in Form der hochreinen freien Base in die Matrix eingebracht wird.

Eine Ausführungsform der Erfindung betrifft eine Vorrichtung zur transdermalen Verabreichung von Fesoterodin, wobei Fesoterodin in einer selbstklebenden Polymerschicht gelöst oder dispergiert ist, mit der Maßgabe, dass für den Fall, dass die selbstklebende Polymerschicht aus Silikonen besteht, in der die freie Base Fesoterodin in Form von Mikroreservoiren dispergiert ist, diese Silikone entweder

(a) in einer Mischung mit anderen nicht-silikonbasierten Polymeren vorliegen oder
(b) in einer Mischung mit Weichmachern vorliegen, wobei die Mischung eine dynamische Viskosität von unter 100 Pa.s und bevorzugt von weniger als 80 Pa.s bei Temperaturen von 200°C aufweist.

Abbildung 3 zeigt den in-vitro Flux durch Mäusehaut, der mit einem im Heißschmelzverfahren hergestellten Silikon-basierten Pflaster, das Ozokerit als Weichmacher für die Klebermatrix enthält und das die hochreine freie Base von Fesoterodin in der Klebermatrix enthält, erreicht wurde.

EVA-Kleber

EVA-Haftkleber sind heißschmelzfähige Haftkleber, die auf Ethylenvinylacetat-Copolymeren beruhen ("EVA-Haftkteber"). Solche EVA-Kleber werden beispielsweise beschrieben in US 4,144,317. EVA-Kleber zeichnen sich aus durch gute Klebeeigenschaften, einfache Herstellung und Verarbeitung sowie gute Hautverträglichkeit. EVA-Kleber können bezogen werden, z.B. bei Beardow Adams (13/BA).

Zur Verarbeitung von EVA-Haftklebern im Heißschmelzverfahren gilt grundsätzlich das unter Silikonen Gesagte, wobei den EVA-Haftklebern keine Weichmacher zugesetzt werden müssen.

Ein Gegenstand der Erfindung ist daher eine Vorrichtung zur transdermalen Verabreichung von Fesoterodin, dadurch charakterisiert, dass Fesoterodin in einer selbstklebenden Polymerschicht (Klebermatrix) vorliegt, wobei die Klebermatrix einen Haftkleber vom EVA-Typ enthält.

Vorzugsweise wird Fesoterodin in Form der freien Base, besonders bevorzugt in Form der hochreinen freien Base, in die EVA-Matrix eingebracht.

In einer besonders bevorzugten Ausführungsform der Erfindung wird die EVA-basierte Klebermatrix im Heißschmelzverfahren hergestellt.

Abbildungen 2 und 3 zeigen die in-vitro Fluxraten durch Humanhaut, bzw. Mäusehaut, die mit einem im Heißschmelzverfahren hergestellten EVA-basierten Pflaster, bei dem die hochreine freie Base von Fesoterodin in die Klebematrix eingebracht wurde, erreicht wurden.

SxS-Haftkleber

SxS Haftkleber können sowohl im lösungsmittelbasierten Herstellverfahren als auch im Heißschmelzverfahren verarbeitet werden. Unter dem Begriff "SxS Haftkleber" werden in der vorliegenden Patentanmeldung Styrol-Block-Copolymer-basierte Kleber verstanden, die nicht-elastomere Styrolblöcke an den Enden und elastomere Blöcke in der Mitte tragen. Die elastomeren Blöcke können beispielsweise aus Polyethylenbutylen, Polyethylenpropylen, Polybutadien, Polyisobutylen oder Polyisopropen bestehen.

Geeignete SxS-Kleber werden beispielsweise in US 5,559,165 oder US 5,527,536 beschrieben und zeichnen sich aus durch gute Klebeeigenschaften, einfache Herstellung und Verarbeitung sowie gute Hautverträglichkeit.

SxS-Haftkleber können sowohl kommerziell bezogen werden (z.B. als Duro Tak 378-3500 bei National Starch & Chemical) als auch mit einem Heißschmelz Extrusions-Equipment bei der Produktion der wirkstoffhaltigen Pflaster selbst hergestellt werden.

Dazu werden beispielsweise entsprechende Mengen (wenigstens folgender Bestandteile) eines Styrol-Block-Copolymers (z.B. Shell Kraton GX1657 oder Kraton D-1107CU) mit einem aliphatischen und/oder aromatischen Harz (z.B. Keyser Mackay Regalite R1090 oder Regalite R1010 oder Regalite R1100) und einem Öl (z.B. Shell Ondina 933 oder Ondina 941) aus den einzelnen Dosierstationen in den Extruder dosiert, dort vermischt und aufgeschmolzen. Im letzten Schritt wird in den so hergestellten Haftkleber der Wirkstoff in den Extruder eindosiert und die Masse auf Folien laminiert. Typische exemplarische Gewichtsanteile Polymer: Harz: Öl sind z.B. 100:120:20 oder 100:200:50. Durch Variation dieser Mengenanteile können die Eigenschaften des SxS-Haftklebers jeweils an die gewünschten Eigenschaften des TTS (Klebkraft, minimaler Kaltfluß, Klebezeitdauer, Freisetzungsprofil des Wirkstoffes, etc.) angepaßt werden.

Ein Gegenstand der Erfindung ist daher eine Vorrichtung zur transdermalen Verabreichung von Fesoterodin, dadurch charakterisiert, dass Fesoterodin in einer selbstklebenden Polymerschicht (Klebermatrix) vorliegt, wobei die Klebermatrix einen Haftkleber auf SXS-Basis umfasst.

Vorzugsweise wird Fesoterodin in Form der freien Base, besonders bevorzugt in Form der hochreinen freien Base, in die SXS-Matrix eingebracht.

Besonders bevorzugt wurde in die erfindungsgemäße SXS-basierte Matrix als Wirkstoff die freie Base, ganz besonders bevorzugt die hochreine freie Base von Fesoterodin eingebracht.

In einer besonders bevorzugten Ausführungsform der Erfindung wird die SXS-basierte Klebermatrix im Heißschmelzverfahren hergestellt.

Abbildungen 2 und 3 zeigen die in-vitro Fluxraten durch Humanhaut, bzw. Mäusehaut, die mit einem im Heißschmelzverfahren hergestellten SxS-basierten Pflaster, bei dem die hochreine freie Base von Fesoterodin in die Klebematrix eingebracht wurde, erreicht wurden.

Wegen der potentiell oxidativen Wirkung der SXS-Kleber, werden SXS-basierten Klebermatrices bevorzugt Antioxidanzien zugesetzt. Ein Beispiel für ein kommerziell erhältliches, geeignetes Antioxidanz ist Irganox$^{R}$ (CIBA).

<u>Acrylatkleber:</u>

Polyacrylate werden durch Radikal-Polymerisation von (Meth)Acrylsäure Derivaten produziert, wobei andere geeignete Verbindungen, wie z.B. Vinylacetat als weitere Monomere benutzt werden können. Zur Klarstellung sei gesagt, dass der hier gebrauchte Ausdruck "Polyacrylat" Polymere beinhaltet, die Einheiten umfassen, die auf Acrylsäure und/ oder Meth-Acrylsäure beruhen sowie Copolymere und Mischungen davon.

Bei der Auswahl von geeigneten Monomeren können die daraus resultierenden Haftkleber prinzipiell so dargestellt werden, dass sie spezifische Eigenschaften aufweisen, d.h. eine günstige Lösungskapazität für den Wirkstoff, eine gewünschte Beweglichkeit des Wirkstoffes in der Matrix sowie eine gewünschte Transfer-Rate über die Haut. Die Transfer-Rate wird wesentlich beschränkt durch den Distributions-Koeffizienten und die Resorption des Wirkstoffes durch die Haut.

Der drucksensitive Haftkleber des Polyacrylat-Typs kann ein Homopolymer und/oder Copolymer von mindestens einem Acrylsäure- und/oder Meth-Acrylsäure-Derivat in Form einer Lösung in einem organischen Lösungsmittel sein. Der Polyacrylat-Typ Haftkleber kann quervernetzbar oder nicht-quervernetzbar sein. Das quervernetzende Reagens verbindet die Polymerketten mittels reaktiver Gruppen mit dem Resultat erhöhter Cohäsion des Haftklebers.

Bevorzugt besteht der Polymer-Haftkleber des Polyacrylat-Typs mindestens aus den folgenden Monomeren:

Acrylsäure, Acrylamid, Hexyl-Acrylat, 2-Ethyl-Hexyl-Acrylat, Hydroxy-Ethyl-Acrylat, OctylAcrylat, Butyl-Acrylat, Methyl-Acrylat, Glycidyl-Acrylat, Methyl-Acrylat, Methacrylsäure, Methacrylamid, Hexyl-Methacrylat, 2-Ethyl-Hexylamid-Acrylat, Octyl-Methacrylat, MethylMethacrylat, Glycidyl-Methacrylat, Vinylacetat, Vinylpyrrolidon, Allyl-Acrylat.

Bevorzugt sind die Polymer Haftkleber des Acrylat-Typs quervernetzbare Haftkleber, die aus einer Kombination der folgenden Monomere polymerisiert werden:

2-Ethyl-Hexyl-Acrylat/N-Butyl-Acrylat/Butyl-Acrylat/Acrylsäure,
2-Ethyl-Hexyl-Acrylat/N-Butyl-Acrylat/Ninylacetat/Acrylsäure,
2-Ethyl-Hexyl-Acrylat/Vinylacetat/Acrylsäure,
2-Ethyl-Hexyl-Acrylat/Vinylacetat/Allyl-Acrylat,
2-Ethyl-Hexyl-Acrylat/Vinylacetat/Divinyl-Benzol/Acrylsäure,
2-Ethyl-Hexyl-Acrylat/Vinylacetat/Allyl-Methacrylat/Acrylsäure,
2-Ethyl-Hexyl-Acrylat/Vinylacetat/2-Hydroxy-Ethyl-Acrylat,
2-Ethyl-Hexyl-Acrylat/Vinylacetat/2-Hydroxy-Ethyl-Methacrylat,
2-Ethyl-Hexyl-Acrylat/Fumarsäure-Diethyl-Ester/Acrylsäure,
2-Ethyl-Hexyl-Acrylat/Maleinsäure-Diethyl-Ester/2-Hydroxy-Ethyl-Acrylat.

Als bevorzugte quervernetzbare Mittel können die folgenden Verbindungen genannt werden: Diphenyl-Methan-4-Diisocyanat, Hexamethylen-Diisocyanat, Titanium-Acetylacetonat, Aluminium-Acetylacetonat, Eisen-Acetylacetonat, Zink-Acetylacetonat, Magnesium-Acetylacetonat, Zirkonium-Acetylacetonat, 2-Ethyl-1,3-Hexanediol-Titanat, Tetra-Isooctyl-Titanat, Tetra-Nonyl-Titanat, polyfunktionale Propylen-Imin-Derivate, Ether-Derivate von Melamin-Formaldehyd-Harze, hoch methylierte Urethan-Harze, Imino-Melamin-Harze.

Die nicht-quervernetzbaren Haftkleber können bevorzugt aus einer Kombination der folgenden Monomere polymerisiert werden:

2-Ethyl-Hexyl-Acrylat/N-Butyl-Acrylat/Vinylacetat,
2-Ethyl-Hexyl-Acrylat/Vinylacetat,
2-Ethyl-Hexyl-Acrylat/N-Butyl-Acrylat/Vinylacetat/Allyl-Acrylat,
2-Ethyl-Hexyl-Acrylat/N-N-Butyl-Acrylat/Allyl-Methacrylat,
2-Ethyl-Hexyl-Acrylat/N-Butyl-Acrylat/Vinylacetat/Divinyl-Benzol,
2-Ethyl-Hexyl-Acrylat/Fumarsäure-Diethyl-Ester/Allyl-Acrytat,
2-Ethyl-Hexyl-Acrylat/Maleinsäure-Diethyl-Ester/Allyl-Acrylat,
2-Ethyl-Hexyl-Acrylat/N-Butyl-Acrylat/Acrylamid/Vinylacetat/Allyl-Acrylat,
2-Ethyl-Hexyl-Acrylat/N-Butyl-Acrylat/Iso-Butyl-Acrylat/Vinylacetat/Allyl-Acrytat.

Darüber hinaus können einige Haftkleber in Form wässriger Dispersion (Dispersionstyp) verwendet werden. Der Gebrauch dieser Dispersionstyp Haftkleber kann den Vorteil bringen, dass während des Überziehens und Trocknens keine brennbaren oder toxischen Lösungsmittel verdampfen.

Dispersionstyp Haftkleber können bevorzugt polymerisiert werden aus einer Kombination der folgenden Monomere:

N-Butyl-Acrylat/Iso-Butyl-Acrylat/Acrylsäure.
2-Ethyl-Hexyl-Acrylat/N-Butyl-Acrylat/Acrylsäure,
2-Ethyl-Hexyl-Acrylat/N-Butyl-Acrylat/2-Hydroxy-Ethyl-Acrylamid,
2-Ethyl-Hexyl-Acrylat/N-Butyl-Acrylat/Vinylacetat/Acrylamid,
2-Ethyl-Hexyl-Acrylat/N-Butyl-Acrylat/Vinylacetat/2-Hydroxy-Ethyl-Acrylat,
2-Ethyl-Hexyl-Acrylat/N-Butyl-Acrylat/Allyl-Acrylat/Acrylsäure,
2-Ethyl-Hexyl-Acrylat/N-Butyl-Acrylat/Vinylacetat/Divinyl-Benzol.

Geeignete Polyacrylate zur Verwendung in der vorliegenden Erfindung werden durch mehrwertige Metall-Ionen quervernetzt, um die physikalischen Eigenschaften des Haftklebers zu verbessern oder um ihn an die spezifischen Anforderungen anzupassen. Die Metall-Ionen werden normalerweise in Form von Metall-Chelaten angewandt, welche in organischen Lösungsmitteln löslich sind. Besonders geeignete quervernetzende Mittel sind Aluminium-Acetyl-Acetonat und Titanium-Acetyl-Acetonat.

Falls der gemäß der vorliegenden Erfindung verwendete Haftkleber ein Polyacrylat-Haftkleber ist, hängt die Löslichkeitskapazität generell vom Typ und der Menge der freien funktionalen Gruppen in dem Haftkleber ab.

Die zur Verwendung in der Vorrichtung der vorliegenden Erfindung am meisten bevorzugten Haftkleber sind Polyacrylate mit polaren Gruppen, insbesondere mit freien Hydroxygruppen. Beispiele solcher Haftkleber sind Polyacrylate, zu deren Herstellung polare Monomere, wie z.B. Hydroxy-Ethyl-Acrylat, Hydroxy-Ethyl-Methacrylat, Acrylsäure oder Methacrylsäure in einer Menge von ca. 1-10 % (w/w), besonders bevorzugt in einer Menge von 3-8% (w/w), ganz besonders bevorzugt in einer Menge von 4-6 % (w/w) verwendet werden. Solche Haftkleber sind im Handel erhältlich unter dem Markennamen Duro-Tak® (National Starch & Chemicals; Hamburg).

Ganz besonders bevorzugt zum Gebrauch in der Vorrichtung der vorliegenden Erfindung sind Haftkleber des Polyacrylat-Typs, wobei Hydroxy-Ethyl-Acrylat und/oder Hydroxy-Ethyl-Methacrylat Monomere während der Polymerisation in einer Menge von 3-8 % (w/w), ganz besonders bevorzugt in einer Menge von 4-6 % (w/w) beigemischt werden.

Solch ein Haftkleber kann entsprechend dem allgemeinen Verfahren erhalten werden, das im US Patent 5.498,418 wie folgt beschrieben wird: Der Haftkleber kann erhalten werden durch radikalische Polymerisation. Im ersten Schritt wird eine Mischung, bestehend aus 21 bis 40 Gew.-% Vinylacetat, 55 bis 70 Gew.-% eines Acrylsäure-$C_{2-8}$-Alkyl-Esters und 3 bis 10 Gew.-% eines Acrylsäure-$C_{2-4}$-Hydroxyl-Acryl-Esters, mit 100 Gew.-% Monomeren in der Mischung, in einem organischen Lösungsmittel hergestellt.

Im zweiten Schritt wird ein konventionelles quervernetzendes Mittel in einem organischen Lösungsmittel und - optional - der Wirkstoff in der für den beabsichtigten Gebrauch der transdermalen Vorrichtung (Pflaster) geforderten Qualität beigemischt, falls notwendig in einem organischen Lösungsmittel.

Schließlich wird in einem dritten Schritt die erhaltene Mischung des besonderen Arylat-Vinylacetat-Copoloymers in einer zusätzlichen Stufe quervernetzt, begleitet durch Erhitzen und durch Entfernen des organischen Lösungsmittels oder der Mischung von verwendeten Lösungsmitteln. Der erhaltene Wirkstoff wird in die Haftklebersubstanz auf besondere Weise durch die aufeinanderfolgende und zusätzliche Quervernetzung des speziellen Acrylat-Vinylacetat-Copolymers "eingebaut".

Alternativ kann das Acrylat-Vinylacetat-Copolymer in Abwesenheit des Wirkstoffes polymerisiert und quervernetzt werden. Der Wirkstoff wird dann erst während der Verwendung des Acrylat-Vinylacetat-Copolymers bei der Pflasterherstellung zugesetzt. Das Acrylat-Vinylacetat-Copolymer hat eine relative Viskosität von 3,0 bis 4,2 bei 20° C.

Bevorzugt enthält die Mischung von Monomeren 2-Ethylhexylacrylat und Hydroxyethylacrylat zusätzlich zu Vinylacetat. Bevorzugt wird die anschließende Quervernetzung des speziellen Acrylat-Vinylacetat-Copolymers mit einem

Titanium-Säure-Ester bestehend aus Polybutyl-Titanat und/oder Titanium-Acetylacetonat, durchgeführt, bevorzugt in einer Menge von 0,3 bis 3 Gew.-% im Verhältnis zum Gewicht des Copolymers.

Ein Prozess zur Herstellung eines TTS gemäß dieser Erfindung kann die folgenden Schritte umfassen: Als ersten Schritt Herstellung einer Lösung eines Copolymers, in welchem optional der Wirkstoff in der für den beabsichtigten Gebrauch des TTS erforderlichen Menge sowie ein konventioneller Quervernetzer oder eine Mischung davon enthalten ist, und wobei das Copolymer erhalten wird durch die radikale Polymerisation einer Mischung von Monomeren bestehend aus 21 bis 40 Gew.-% Vinylacetat, 55 bis 70 Gew.-% einer Acrylsäure-$C_{2-8}$-Alkyl-Ester und 1 bis 10 Gew.-% einer Acrylsäure-$C_{2-4}$-Hydroxyalkylester, Auftrag der oben genannten Lösung in der erforderlichen Schichtdicke auf dem Schutzfilm des TTS und Entfernen des Lösungsmittels oder Mischung von Lösungsmitteln durch Erhitzen, was sich in einer zusätzlichen Quervernetzung des speziellen Acrylat-Vinylacetat-Copolymers auswirkt.

Eine Ausführungsform solch eines Prozesses ist darin charakterisiert, dass das Acrylat-Vinylacetat-Copolymer, - optional - der Wirkstoff und das quervernetzbare Mittel anfangs in einem Lösungsmittel aufgelöst werden, welches 20 bis 40 Gew.-% Ethanol oder eine Ethanol-Methanol-Mischung enthält, mit einem Verhältnis von festen Bestandteilen bestehend aus 40 bis 60 Gew.% der Mischung des speziellen Acrylat-Vinylacetat-Copolymers, des quervernetzbaren Mittels und des Wirkstoffes.

In einer anderen - bevorzugten - Ausführungsform der Erfindung wird der Wirkstoff erst nach Quervernetzung des Acrylats zur Dispersion gegeben, die dann nach Homogenisierung auf den Schutzfilm aufgetragen wird.

Ein besonderes Ausführungsbeispiel für die Zubereitung solch eines Acrylat-Vinylacetat-Haftklebers wird offenbart in US-A-5,498,418, Spalte 2, Zeile 61 bis Spalte 3, Zeile 10. Dieses Dokument wird hier als Referenz angeführt.

Ein besonders bevorzugter Haftkleber zum Gebrauch in der vorliegenden Erfindung sind die im Handel erhältlichen Haftkleber Duro-Tak® 387-2287 und Duro-Tak® (3)87-4287 (National Starch & Chemicals; Hamburg). In einer besonders bevorzugten Ausführungsform der Erfindung wird der Duro-Tak-Haftkleber in geeignetem Lösungsmittel mit der gewünschten Menge des Wirkstoffes gemischt und die resultierende homogene Dispersion in gewünschter Dicke ausgestrichen. Schließlich wird das Lösungsmittel oder das Lösungsmittelgemisch bei erhöhten Temperaturen (50 - 70°C) entfernt.

Ein Gegenstand der Erfindung ist daher eine Vorrichtung zur transdermalen Verabreichung von Fesoterodin, dadurch charakterisiert, dass Fesoterodin in einer Polymerschicht, vorzugsweise in einer selbstklebenden Polymerschicht (Klebermatrix) vorliegt, wobei die Polymerschicht mindestens ein Polymer vom Acrylat und/oder Methacrylat-Typ umfasst.

Vorzugsweise wird Fesoterodin in Form der freien Base, besonders bevorzugt in Form der hochreinen freien Base, in die Acrylat-Matrix eingebracht.

Abbildung 3 zeigt die in-vitro Fluxraten durch Mäusehaut, die mit einem im Heißschmelzverfahren hergestellten Acrylat-basierten Pflaster, bei dem die hochreine freie Base von Fesoterodin in die Klebematrix eingebracht wurde, erreicht wurde.

<u>Hilfs- und Zusatzstoffe</u>

Die oben beschriebenen wirkstoffhaltigen Polymermatrices der erfindungsgemäßen transdermalen Vorrichtungen können weitere Hilfs- und Zusatzstoffe enthalten. Beispiele sind Puffer, Lösungsvermittler, Kristallisationsinhibitoren, chemische Stabilisierungsmittel, Antioxidanzien, weitere Hilfsmittel zur Retardierung sowie Hautpenetrationsenhancer.

Hautpenetrationsenhancer können beispielsweise zugesetzt werden, um die Wirkstoffmenge, die durch die Haut permeiert, zu vergrößern oder die Auftragsfläche der Vorrichtung zu verkleinern. Beispiele für gängige Penetrationsenhancer sind: Alkohole, insbesondere kurzkettige Alkohole wie Ethanol. Fettalkohole z.B. Laurylalkohol, Polyalkohole wie Glycerin; Amide, z.B. aromatische Amide wie N,N-Diethyl-m-Toluamid; Aminosäuren; Azone; Öle, wie z.B. Menthol oder Pfefferminzöl; Fettsäuren und deren Ester, wie z.B. Ölsäure, Laurylsäure, Isopropylmyristat oder Glycerolmonolaurat; Macrocylen, wie z.B. Cyclopentadecanon; Phospholipide wie z.B. Lecithin; 2-Pyrrolidone sowie Sulfoxide, wie z.B. Dimethylsulfoxid.

Auf Grund der guten Penetrationseigenschaften von Fesoterodin werden Ausführungsformen der Erfindung bevorzugt, in denen auf die Zugabe eines Enhancers verzichtet wird.

Als weitere Bestandteile kann der Klebermatrix eine hydrophile Komponente, wie z.B. ein hydrophiles Polymer zugesetzt werden. Diese hydrophilen Polymere können als Löslichkeitsvermittler bzw. Kristallisationsinhibitoren für Fesoterodin dienen und zu einer gleichmäßigen Verteilung des Wirkstoffs in der Klebermatrix beitragen.

Geeignete hydrophile Polymere zur Verwendung im erfindungsgemäßen TTS können beispielsweise ausgewählt sein aus der Gruppe der Polysaccharide, substituierte Polysaccharide, Polyethylenoxide, Polyvinylacetate, Polyvinylpyrrolidone (PVP), PVP mit geeignetem Weichmacher, Polyethylenglycole, Polypropylenglycole, Polyacrylate, Copolymere aus Polyvinylpyrrolidon und (Poly)vinylacetat, Copolymere von Ethylen und Vinylacetat sowie Polyvinylalkoholen mit geeignetem Weichmacher, z.B. Glycerin.

Bevorzugte hydrophile Polymere sind PVP, Polyethylenoxide (PEO), Polyvinylacetate (PVAc) sowie Copolymere aus PVP und Vinylacetat.

Die hydrophilen Polymere können der Kleberschicht beispielsweise in einem Anteil von 0,5-40 Gew-% bezogen auf das Gesamtgewicht der Kleberschicht zugesetzt werden. Bevorzugt werden 2-25 Gew-%, besonders bevorzugt 2-15 Gew-% oder 2-10 Gew-% hydrophile Polymere zugesetzt.

Zum Einsatz im Heißschmelzverfahren sind solche hydrophilen Polymere besonders geeignet, die bei Temperaturen unterhalb von 170°C eine dynamische Schmelzviskosität von maximal 150 Pa.s, bevorzugt kleiner als 120 Pa.s und besonders bevorzugt von unter 80 Pa.s aufweisen. Ist die dynamische Viskosität des hydrophilen Polymers bei der gewünschten Verarbeitungstemperatur zu gering, so muss gegebenenfalls ein geeigneter Weichmacher, z.B. Glycerin, vorab zugesetzt werden.

Der Zusatz der oben genannten hydrophilen Polymere kann insbesondere bei sehr hydrophoben Kleber-Matrices, z.B. Silikon-, Polyisobutylen- oder SXS-Matrices, vorteilhaft sein.

Wie bereits in der WO 01/35957 beschrieben, neigen die freien Basen der 3,3-Diphenylpropylamin-Monoester zur Gehaltsabnahme, z.B. durch Hydrolyse und Umesterung. Es wurde nun überraschend festgestellt, dass sich die 3,3-Diphenylpropylamin-Monoester in Matrices mit hydrophilen Anteilen signifikant stabilisieren lassen.

Während beispielsweise die freie Base von Fesoterodin als Öl nach 6-monatiger Lagerung bei 5°C zu ca. 3-4 % zersetzt ist, lässt sich eine Gehaltsabnahme nicht oder nur in wesentlich geringerem Maße feststellen, wenn Fesoterodin in Matrices eingearbeitet ist, die polare Bestandteile enthalten.

Beispiele für solche Matrices, die zur Stabilisierung von Fesoterodin führen, sind beispielsweise Matrices, die Polyacrylate, insbesondere Polyacrylate mit polaren Gruppen, EVA oder Mischungen von Silikonklebern mit hydrophilen Polymeren, z.B. PVP, PVAc oder PEO, enthalten (Tabelle 4).

Tabelle 4: Stabilisierung von Fesoterodin in verschiedenen Matrices bei Lagerung

| **Matrix** | **5°C Stabilisierungsfaktor[1]** | **25°C / 60 %RH Stabilisierungsfaktor[1]** | **Herstellverfahren** |
|---|---|---|---|
| EVA | 7-fach | 4,5-fach | Heißschmelz |
| Silikon/Cer[3] | --- | --- | Heißschmelz |
| Silikon + 2 % PVP | 2-fach | 2-fach | Lösemittel |
| Silikon/Cer[3] + 5 % PEO | 3-fach | 2,5-fach | Heißschmelz |
| Polyacrylat | kein Abbau nachweisbar[2] | 13-fach | Lösemittel |
| PIB | --- | --- | Lösemittel |
| SXS | --- | 1,1-fach | Heißschmelz |

[1]Der Stabilisierungsfaktor wurde durch Division der durchschnittlichen monatlichen Gehaltsabnahme von Fesoterodin-Base bei Lagerung als Rohstoff (Öl) durch die durchschnittliche monatliche Gehaltsabnahme bei Lagerung in Matrices bestimmt; [2]bis zum Ende der Meßperiode nach 6 Monaten; [3]Cer=Ceresin

Wie Tabelle 4 zeigt, führt die Einarbeitung von Fesoterodin in Matrices, bestehend aus EVA-Kleber, Polyacrylatkleber oder Mischungen aus Silikon-Kleber mit hydrophilen Polymeren wie PEO oder PVP zu einer deutlichen Stabilisierung von Fesoterodin, und zwar unabhängig vom Herstellverfahren (Heißschmelz- oder Lösemittelverfahren).

Eine Ausführungsform der Erfindung betrifft daher Vorrichtungen, in denen Fesoterodin als freie Base einer langsameren Gehaltsabnahme unterworfen sind, als dies der Fall ist, wenn die freie Base unter identischen Bedingungen nicht in ein Polymer eingebettet als Öl gelagert wird. Bevorzugte Ausführungsformen sind solche, die bei 5°C und/oder bei 25°C zu einer 2-, 3-, 7- oder 10-fachen Stabilisierung des 3,3-Diphenylpropylamin-Monoesters im Vergleich zur Lagerung als freie Base führen.

Besonders bevorzugte erfindungsgemäße Vorrichtungen sind solche, in denen die freie Base in einer Polymerschicht vorliegt, in der eine Gehaltsabnahme von Fesoterodin von weniger als 3%, bevorzugt weniger als 2% oder 1% bei 6-monatiger Lagerung bei 4°C und von weniger als 10%, bevorzugt weniger als 5% und besonders bevorzugt weniger als 3% oder 1,5% bei 3-monatiger Lagerung bei 25°C und 60% Luftfeuchtigkeit auftritt.

Bevorzugte Matrices sind solche, die 50-95 Gew-% eines Haftklebers enthalten, der ausgewählt ist aus der Gruppe der

- Acrylatkleber sowie deren Copolymere, insbesondere Acrylatkleber mit polaren Gruppen, z.B. mit freien Hydroxygruppen,
- EVA-Kleber
- Silikonkleber, die 2-25 Gew-%, bevorzugt 2-10 Gew-% eines hydrophilen Polymers, insbesondere ausgewählt aus

PEO, PVP oder PVAc enthalten,
- SXS- oder PIB-Kleber, die 2-25 Gew-%, bevorzugt 2-10 Gew-% eines hydrophilen Polymers enthalten,
- Mischungen aus hydrophilen Haftklebern (z.B. polaren Polyacrylaten) mit hydrophoben Haftklebern (z.B. Silikon-, SXS- oder PIB-Klebern).

Ganz besonders bevorzugte Haftkleber zur Herstellung der erfindungsgemäßen Matrices sind Polyacrylate, insbesondere solche mit polaren Gruppen. Diese Matrices weisen sowohl ein exzellentes Freisetzungsprofil für Fesoterodin als auch hervorragende Stabilisierungseigenschaften für 3,3-Diphenylpropylamin-Monoester auf.

Die Erfindung betrifft weiterhin die Verwendung der freien Basen von Fesoterodin zur Herstellung kontrolliert freisetzender transdermaler Arzneiformen.

Ein Aspekt der Erfindung ist daher die Verwendung von Fesoterodin zur Herstellung eines Arzneimittels zur transdermalen Verabreichung, dadurch gekennzeichnet, dass Fesoterodin in Form der freien Base zu einer Polymerschicht, vorzugsweise einer selbstklebenden Polymerschicht (Klebermatrix) zugesetzt wird.

Bevorzugt liegt dabei Fesoterodin als freie Base mit einem Reinheitsgrad von wenigstens 98 Gew%, bevorzugt wenigstens 99%, besonders bevorzugt 99,5% und ganz besonders zu wenigstens 99,8% vor.

In einer bevorzugten Ausführungsform wird die freie Base von Fesoterodin zur Herstellung eines transdermalen Arzneimittels verwendet, welches

(a) eine Grundfläche von maximal 40 $cm^2$ aufweist,
(b) eine selbstklebende Polymerschicht umfasst, die

(b1) ein Gewicht von 30-300 $g/m^2$ aufweist,
(b2) 50-95 Gew% eines Haftklebers enthält,
(b3) Fesoterodin in einer Konzentration von 5-40 Gew% bezogen auf das Gesamtgewicht der Polymerschicht enthält,

(c) das Fesoterodin über einen Zeitraum von mindestens 24 Stunden bei einer konstanten Fluxrate von mindestens 125 $\mu$g/h durch Humanhaut abgibt.

Ein typisches und bevorzugtes Beispiel für ein solches bevorzugtes Arzneimittel ist ein sogenanntes monolithisches Pflaster, bestehend aus einer wirkstoffhaltigen Klebermatrix (1), eine für die Inhaltsstoffe der Klebermatrix inerte und undurchlässige Rückschicht (2) sowie eine unmittelbar vor Gebrauch ablösbare Schutzschicht (3) umfasst (Figur 4).

Wie aus Abbildung 2 und Tabelle 3 ersichtlich ist, lassen sich durch die erfindungsgemäße Verwendung der freien Base von Fesoterodin transdermale Arzneimittel herstellen, die bei Beladung mit 15 Gew% Wirkstoff und einer Oberfläche von 20 $cm^2$ Fesoterodin in einer Fluxrate von 6-8 mg pro Tag durch Humanhaut transportieren. Durch eine entsprechende Variation der Oberfläche von 5-50 $cm^2$ ist somit in einfacher Weise und ohne Rezepturänderung ein täglicher Wirkstofflux zwischen 0,5 und 20 mg über einen Zeitraum von mindestens 1 oder 2 Tagen möglich (Tabelle 3).

Durch die Variation der Wirkstoffkonzentration und der Beladung der erfindungsgemäßen Vorrichtung ist zudem eine weitere Anpassung der Wirkstoffanflutung und/oder eine Kontrolle der Freisetzungsdauer möglich.

Die erfindungsgemäßen Vorrichtungen bzw. Arzneimittel sind besonders zur Behandlung von Inkontinenz, insbesondere von Dranginkontinenz, Hyperaktivität des Detrusors, Pollakisurie, Nykturie oder imperativem Harndrang geeignet.

Die Erfindung betrifft ferner die Herstellung der erfindungsgemäßen Vorrichtungen zur transdermalen Verabreichung.

Ein weiterer Gegenstand der Erfindung ist eine Methode zur Vorbeugung oder/und Behandlung von Inkontinenz, insbesondere Dranginkontizenz, Hyperaktivität des Detrusors, Pollakisurie, Nykturie oder imperativem Harndrang durch die Verabreichung von Fesoterodin, wie vorstehend beschrieben, und/oder durch Verabreichung einer erfindungsgemäßen Vorrichtung, enthaltend Fesoterodin, auf die Haut eines Säugers, insbesondere auf die Haut eines Menschen, der der Vorbeugung oder Behandlung der vorgenannten Erkrankungen bedarf.

**Ausführungsbeispiele:**

**1. Herstellung der hochreinen freien Base von Fesoterodin**

A. <u>Herstellung der Base Fesoterodin (B. s. Abbildung 1. R= i-Pr)</u>

Zu einer auf -3° C gekühlten Lösung von 59,8 g (175,1 mol) (R)-2-[3-(Diisopropylamino)-1-phenylpropyl]-4-(hydroxymethyl)phenol (A, s. Abbildung 1) gelöst in 750 ml Dichlormethan wurde unter Rühren und Eisbadkühlung eine

Lösung von 18,6 g Isobuttersäurechlorid in 250 ml Dichlormethan in ca. 10 Minuten zugetropft. Nach ca. 5 Minuten fiel weiße Substanz aus. Hierzu wurde unter Rühren und Eisbadkühlung eine Lösung von 17,7 g Triethylamin in 250 ml Dichlormethan in 5 Minuten zugetropft. Der Ansatz wurde nacheinander je einmal mit 250 ml Wasser, 250 ml ca. 5 %ige wässrige $NaHCO_3$ Lösung und 250 ml Wasser gewaschen. Der über $Na_2SO_4$ getrocknete Dichlormethanextrakt wurde am Rotationsverdampfer bis zur Gewichtskonstanz eingeengt, wobei ein blaßgelbes, zähflüssiges Öl übrigblieb.
Rohausbeute: 63,7g (88,5% der Theorie).

Die Reinheit von B in der HPLC betrug in diesem Beispiel 94,1 %. (Typischer Bereich für B: 90.5 % - 94.4 %).

B. Herstellung des Fumaratsalzes (E; Abbildung 1; R = i-Pr. X = Hydrogenfumarat) von Fesoterodin

Eine Lösung von 41.87 g (102 mmol) (R)-2-(3-Diisopropylamino-1-phenylpropyl)-4-hydroxymethylphenylisobuttersäureester (B) in 90 ml 2-Butanon wurde unter Erwärmen mit Fumarsäure (11.81 g, 102 mmol) versetzt. Nach dem Lösen der Säure wurde langsam unter Rühren Cyclohexan (20-30 ml) bis zum Einsetzen einer Trübung zugesetzt. Man beließ den farblosen, homogenen Ansatz zunächst 18 Stunden bei Raumtemperatur, dann mehrere Stunden bei 0° C. Die ausgefallenen farblosen Kristalle wurden abgesaugt, mit wenig Cyclohexan/2-Butanon (90:10, Vol.-%) gewaschen und im Vakuum bei 30° C getrocknet.

Ausbeute: 44.6 g (83.1 % der Theorie) des Hydrogenfumarat-Salzes (E) des (R)-2-(3-Diisopropylamino-1-phenylpropyl)-4-hydroxymethylphenylisobuttersäureester in Form farbloser Plättchen.

Schmp. 98.8° C, eine zweite Kristallisation aus dem gleichen Lösungsmittelgemisch ergab das Produkt mit einem Schmp. von 103° C.
$[\alpha]_D^{20}$= + 6,0 (c = 1,0, Ethanol); - 19,3 (c =1,0, Acetonitril).
$^1$H-NMR ($CDCl_3$): u.a. 6,84 ppm für C*H*= vom Hydrogenfumarat Anion.
$^{13}$C-NMR ($CDCl_3$): u.a. 135,58 ppm und 170,56 ppm für Olefin- und Carbonylkohlenstoffe vom Hydrogenfumarat-Anion.

Die Reinheit in diesem Beispiel an E (bestimmt mit HPLC) betrug 99.2 %.

C. Herstellung der hochreinen Base Fesoterodin (B; Abbildung 1; R= i-Pr).

250 g (0,474 mol) kristallines (R)-2-[3-(Diisopropylamino)-1-phenylpropyl]-4-(hyhdroxymethyl)-phenyl-2-methylpropanoat-Fumarsäuresalz (E) wurde unter Rühren zu 1 l Wasser gegeben und auf 30° C erwärmt. Nach ca. 30 Minuten lag eine fast klare Lösung vor. Zu der auf RT abgekühlten Lösung wurden unter Rühren in ca. 10 Minuten 96,0 g Natriumhydrogencarbonat portionsweise gegeben. Zur fast klaren, farblosen Lösung wurde 1 l Dichlormethan gegeben. Nach einiger Zeit Rühren bei RT (starke $CO_2$-Entwicklung) wurde die Dichlormethanphase abgetrennt und nacheinander je einmal mit 0,2 Liter 5 %-iger wässriger Natriumhydrogencarbonatlösung und 0,2 l Wasser gewaschen. Die filtrierte klare, farblose Dichlormethanphase wurde am Rotavapor bei einer Badtemperatur von ca. 40° C bis zur Gewichtskonstanz eingeengt, wobei zuletzt Membranpumpenvakuum (Endvakuum 5 mbar) angelegt wurde. Dabei blieb (R)-2-(3-Diisopropylamino-1-phenylpropyl)-4-hydroxymethylphenylisobuttersäureester (B) als ein fast farbloses viskoses Öl übrig.
Ausbeute: 180,6 g (92,6% der Theorie)
$[\alpha]_D^{20}$ = + 5,9 (c = 1,0, Ethanol); - 6,7 (c = 1,0, Acetonitril)
NMR ($CDCl_3$): 19,01, 19,95, 20,59, 21,12, 34,28, 36,89, 41,88, 42,32, 43,90, 48,78, 64,68, 122,57, 125,59, 126,16, 126,86, 127,96, 128,54, 136,88, 138,82, 143,92, 147,90, 175,69 (ppm).

Die Reinheit in der HPLC betrug in diesem Beispiel 99,0 %. Typische Reinheiten liegen zwischen 98,7% und 99,5%.
$^1$H- und $^{13}$C-NMR: Keine Resonanzsignale für das Hydrogenfumarat-Anion nachweisbar (vgl. E)

Die Langzeit-Lagerung erfolgt bevorzugt im Dunkeln unter Argon bei -20°C.

D. Herstellung des Hydrogencarbonatsalzes (E: Abbildung 1: R = i-Pr. X = Hydrogencarbonat)

Fesoterodin (107,7 mg, (R)-2-(3-Diisopropylamino-1-phenylpropyl)-4-hydroxymethy)-phenylisobuttersäureester, B) wird mit destilliertem Wasser überschichtet und bei Raumtemperatur gerührt. Nach zwei Tagen Rühren bleibt der Reaktionsansatz unverändert zweiphasig. In der oberen, wässrigen Phase lässt sich dünnschichtchromatographisch kein organisches Material (B oder E) nachweisen (Kieselgel, Laufmittelsystem Petrolether/Aceton/Triethylamin, 70/20/10 Vol.-%).

In den zweiphasigen Reaktionsansatz wird bei Raumtemperatur unter Rühren ein leichter Strom Kohlendioxidgas eingeleitet. Nach zwei Tagen hat sich die untere ölige Phase (Fesoterodin) vollständig und klar in der wässrigen Phase gelöst.
$^{13}$C-NMR-Spektrum des Hydrogencarbonatsalzes von Fesoterodin (δ-Werte):
14,11, 15,36, 15,51, 29,32, 31,09, 38,95, 43,31, 52,38, 60,45, 120,04, 124,07, 124,33, 124,83, 126,12, 131,97, 136,55,

139,06, 144,60, 157,46 (H$\underline{C}$O$^3$), 175,75.

Es ergibt sich eine gute Übereinstimmung mit dem $^{13}$C-NMR-Spektrum des Hydrochlorids von Fesoterodin, hergestellt durch Auflösen der Base in 1 M wässriger Salzsäure (δ-Werte):

13,26, 15,32, 15,48, 29,29, 31,06, 38,95, 43,34, 52,42, 60,49, 120,10, 124,18, 124,38, 124,85, 126,13, 131,97, 136,50, 139,02, 144,61, 175,94.

**2. Herstellung der TTS-Matrices**

2.1. Herstellung einer silikonbasierten Matrix im Heißschmelzverfahren

8,5 g einer silikonbasierten Haftklebermischung aus dem Silikonkleber Bio-PSA 7-4300 (Dow Coming, Michigan) mit 5 Gew.-% Ozokerit oder Ceresin (bezogen bei Dow Corning) wurde für etwa 20 Minuten auf 150°C erhitzt, bis eine homogene Schmelze entstand. 1.5 g Fesoterodin (hochreine freie Base) wurde zugesetzt und das Gemisch für weitere 5 Minuten bei 150°C gehalten. Die Mischung wurde dann manuell homogenisiert und auf eine vorgewärme Folie (120°C, Spaltweite 250 μm) laminiert. Für die Freisetzungstests wurden 5 cm$^2$ Stücke ausgeschnitten.

2.2. Herstellung einer acrylatbasierten Matrix im Lösemittelverfahren

1,5 g hochreine Fesoterodin-Base wurde in Dichlormethan gelöst und zu einer Lösung von 8,5 g DuroTak$^R$ 387-2287 (in Ethylactetat) gegeben. Die resultierende Mischung wurde gerührt, bis eine homogene Dispersion erreicht wurde. Die Dispersion wurde dann auf Folie ausgestrichen und getrocknet (Erichsen 100μm, 6mm/sec, Trocknungszeit: 30 Min bei 50°C).

2.3. Herstellung einer SXS-basierten Matrix im Heißschmelzverfahren

100 Teile SIS (Styrol-Polyisobutylen-Styrol; Kraton D1107CU), 150 Teile Regalite R 1090, 20 Teile Ondinaöl und 1 Teil Irganox wurden Hitze bei 140°C gemischt und aufgeschmolzen. Zu je 8,5 g Schmelze wurden 1.5 g Fesoterodin (hochreine freie Base) zugesetzt und das Gemisch für weitere 1-5 Minuten bei 140°C gehalten. Die Mischung wurde dann mechanisch homogenisiert und auf eine vorgewärme Folie (120°C, 250 μm) laminiert. Es wurden Stücke gewünschter Größe ausgeschnitten.

2.4. Herstellung einer EVA-basierten Matrix im Heißschmelzverfahren

8,5 g des EVA-Heißschmelzklebers wurden für etwa 20 Minuten bei 160°C erhitzt bis eine homogene Schmelze erhalten wurde. 1,5 g bzw. 1,65 g hochreine Fesoterodin-Base wurde dazugegeben und die Mischung sodann manuell homogenisiert. Die Mischung wurde dann auf eine vortemperierte (120°C) Chill-Roll laminiert. Es wurden jeweils 5 cm$^2$ (für Permeationsexperimente) ausgeschnitten.

**3. Freisetzungsexperimente**

3.1. Bestimmung des Wirkstoffflusses im Mäusehautmodell

Für die Fluxmessungen durch Mäusehaut wurde Bauch und Rückenhaut einer Dicke von ca. 120 bis 150 μm in einer horizontalen Diffusionszelle verwendet. Medium: PhosphatPufferlösung (0,066 molar) pH 6,2; 32°C

Die Wirkstoffreisetzung wurde per HPLC bestimmt.

3.2. Bestimmung des Wirkstofffluxes im Humanhautmodell

(a) experimentelles Design

Die Bestimmung des Fesoterodinfluxes durch Humanhaut wurde im wesentlichen durchgeführt wie in H. Tanojo et al, J. Control Rel. 45 (1997) 41-47 beschrieben, wobei anstelle der Silikonmembran eine Dialysemembran [Diachema Dialysemembran, Typ 10.14, bezogen bei Fa. Dianorm, München, DE; hergestellt aus neutraler Zellulose, Ausschlußgröße 5000 Da, Dicke (trocken): 25 μm; Vorbehandlung gemäß Herstellerangaben] verwendet wurde.

Humanhaut wurde in einer Dicke von ca. 250 μm aus dem Abdomen gewonnen. Ein TTS mit einer Fläche von 2,545 cm$^2$ wurde auf Humanhaut gleicher Fläche aufgebracht, wobei die Haut zur Akzeptorseite hin auf einer Silikonmembran aufliegt (Schema 1). Als Akzeptorphase wurde PBS (0,066 molar) bei PH 6,2 und einer Temperatur von 32±0.5°C verwendet. Die Experimente wurden mit einem Flux von 5mL/h über 72 Stunden durchgeführt, wobei alle 3

Stunden Proben entnommen wurden. Zu den Probenentnahmezeiten wird das Freisetzungsmedium gegen frisches, auf 32±0.5°C thermostatiertes Medium ausgetauscht und die Menge des freigesetzten Fesoterodins per HPLC gemessen.

Die Bestimmung der Fluxrate Q(t) erfolgte bezogen auf die Fläche der Meßzelle (0.552 $cm^2$) gemäß der Formel:

$$Q(t) = \mu g/cm^2 = \text{Konzentration Fesoterodin x Volumen des Akzeptors}/0.552\ cm^2$$

**Schema 1:**

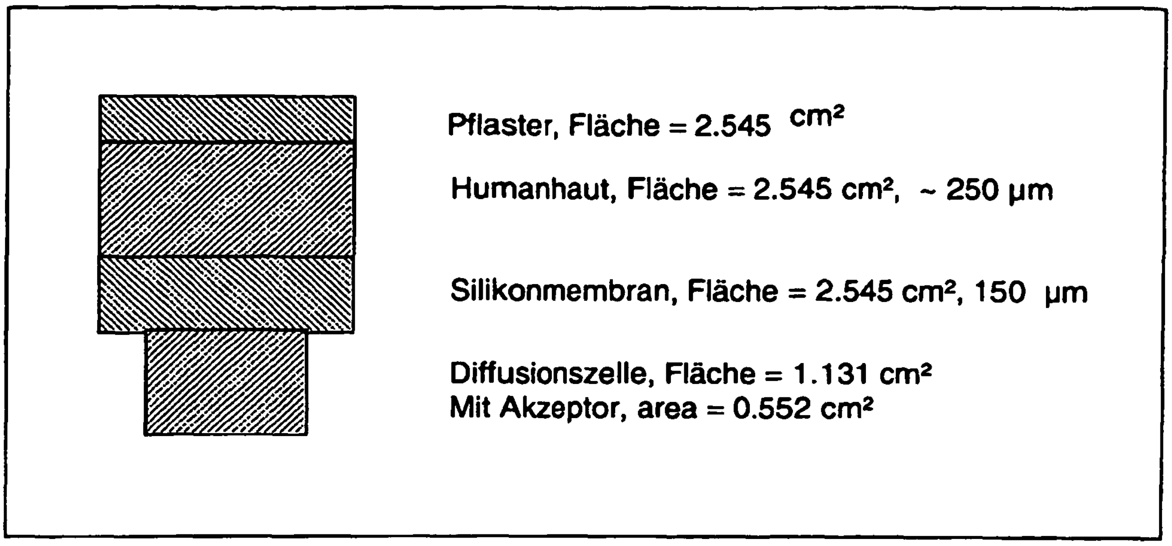


(b) Analytik der Wirkstofffreisetzung

Die Messung des Wirkstoffflux es durch Hautpräparate erfolgte per HPLC (Säule Spherisorb 5CN 25cm) unter folgenden Bedingungen: 4 Volumenteile Acetonitril / 6 Volumenteile $H_2O$ / 0,1% Volumenteile TFA; 35°C, 225 nm, 1ml Fluss

**4. Analytik: Bestimmung der Reinheit des Wirkstoffs**

Zur Bestimmung der chemischen Reinheit von Fesoterodin wurde eine HPLC-Methode verwendet, die auf der Trennung an einer stationären Umkehrphase (reversed phase) beruht und zur Elution einen Lösungsmittelgradienten verwendet.

Materialien (beispielhaft):

Acetonitril für die HPLC, Methansulfonsäure (<99%. Fluka), Wasser (Gereinigt, HPLC-Qualität), Waters Pumpe 510, Säulenofen (Waters Column Heater Modul, 35 °C), Probengeber (Waters Wisp 717, Injektionsvolumen 20 µL), UV-Detektor (Shimatzu SPD 10A). Säule (150x3.9 mm, Symmetry Shield RP8, Waters Part No. WAT 200655).

Mobile Phase:

Acetonitril mit 0,05% Methansulfonsäure (v/v, %), Komponente A Wasser mit 0,05% Methansulfonsäure (v/v, %), Komponente B Gradientenprogramm: Zeit (Min.) 0,0 mit 15% Komponente A und 85% Komponente B, nach 15 Min. 60% A und 40% B, nach 20 Min. 15% A und 85% B. Flußrate: 1,2 ml/Min.

Die Konzentrationen der Referenzlösungen von A, B und C (Abbildung 1/4, R = i-Pr) betrugen 10-250 µg/mL. Bei höheren Konzentrationen trat Tailing mit Peaküberlappung auf.

Auswertung:

Zur Auswertung nach der 100%-Methode wurden die Mittelwerte aller Peakflächen (Dreifachbestimmungen) addiert und gleich 100% gesetzt. Auf diesen Wert wurden die Flächen der einzelnen Peaks (in %) bezogen. Retentionszeiten für A, B und C (Min.): 5,9, 9,0 und 12,6.

**5. Analytik: Bestimmung des Restsalzgehaltes**

Es werden 200 MHz oder 500 MHz $^1$H-NMR-Spektren der freien Base Fesoterodin in $CDCl_3$ als Lösungsmittel aufgenommen und charakteristische Resonanzsignal-Gruppen elektronisch integriert, wie:

$\delta$ = 6,97 ppm (Duplett, aromatischer Wasserstoff, $H^6$, 1H),
$\delta$ = 4,59 ppm (Singulett, HO-C$\underline{H}_2$, 2H),
$\delta$ = 4,10 ppm (Triplett, $H^1$-Propyl, 1 H).

Die Relation zum Resonanzsignal des Anions, z.B. Hydrogenfumarat ($\delta$ = 6.84 ppm, =C$\underline{H}$-, 2H) ergibt den Anteil an Restsalz (in Mol-%).

## Patentansprüche

1. Vorrichtung zur transdermalen Verabreichung von (R)-2-(3-(1,1-Diisopropylamino)-1-phenylpropyl]-4-(hydroxymethyl)phenyl isobutyrat (Fesoterodin), **dadurch** charakterisiert, dass Fesoterodin in einer Polymermatrix vorliegt und in einer Dosis von 0,5-20 mg pro Tag durch Humanhaut freigesetzt wird.

2. Vorrichtung nach Anspruch 1, **dadurch gekennzeichnet, dass** die Vorrichtung hergestellt wird, indem Fesoterodin der Polymermatrix in Form der freien Base zugesetzt wird.

3. Vorrichtung nach einem der vorhergehenden Ansprüche, **dadurch gekennzeichnet, dass** die Polymermatrix 50-95 Gew% eines Haftklebers umfasst und selbstklebend ist.

4. Vorrichtung nach einem der vorhergehenden Ansprüche, **dadurch gekennzeichnet, dass** die Polymermatrix einen oder mehrere Haftkleber umfasst, die ausgewählt sind aus der Gruppe der Acrylate, Ethylenvinylactetate (EVA), Silikone oder Styrolblock-Copolymere (SXS).

5. Vorrichtung nach einem der vorhergehenden Ansprüche, **dadurch gekennzeichnet, dass** die Polymermatrix zu 50-95 Gew% aus einer heißschmelzfähigen Mischung aus einem Silikonhaftkleber und mindestens einem Weichmacher besteht.

6. Vorrichtung nach einem der vorhergehenden Ansprüche, **dadurch gekennzeichnet, dass** die Polymermatrix zu 50-95 Gew% aus (a) einem hydrophilen Haftkleber und/oder (b) einer Mischung eines hydrophoben Haftklebers mit 2-20 Gew%, bezogen auf das Gesamtgewicht der Polymermatrix, eines hydrophilen Polymers und/oder (c) einer Mischung eines hydrophilen mit einem hydrophoben Haftkleber besteht.

7. Vorrichtung nach Anspruch 6, wobei das hydrophile Polymer PEO, PVP oder PVAc ist.

8. Vorrichtung nach einem der vorhergehenden Ansprüche, **dadurch** charakterisiert, dass Fesoterodin in einem Reinheitsgrad von über 97 Gew% in die Polymermatrix eingearbeitet wurde.

9. Vorrichtung nach einem der vorhergehenden Ansprüche, **dadurch gekennzeichnet, dass** die Vorrichtung

    (a) eine Grundfläche von maximal 50 $cm^2$ aufweist,
    (b) eine selbstklebende Polymerschicht umfasst, die

        (b1) ein Gewicht von 30-300 $g/m^2$ aufweist,
        (b2) 50-95 Gew% eines Haftklebers enthält,
        (b3) Fesoterodin in einer Konzentration von 5-40 Gew% bezogen auf das Gesamtgewicht der Polymermatrix enthält und

(c) Fesoterodin über einen Zeitraum von mindestens 24 Stunden bei einer konstanten Fluxrate von mindestens 4 $\mu$g/cm$^2$/Stunde durch Humanhaut abgibt.

**10.** Vorrichtung nach einem der vorhergehenden Ansprüche, **dadurch gekennzeichnet, dass** die Vorrichtung eine Grundfläche von maximal 40 cm$^2$ aufweist und die Wirkstoffbeladung der selbstklebenden Polymermatrix 7-30 Gew% beträgt.

**11.** Vorrichtung nach einem der vorhergehenden Ansprüche, **dadurch** charakterisiert, dass die Vorrichtung eine Verbindung der Formel I in einer Dosis von mindestens 3 mg pro Tag über mindestens 24 Stunden in konstanter Fluxrate durch Humanhaut befördert.

**12.** Vorrichtung nach einem der vorhergehenden Ansprüche, **dadurch** charakterisiert, dass die Vorrichtung eine wirkstoffhaltige Klebermatrix (1), eine für die Inhaltsstoffe der Klebermatrix inerte und undurchlässige Rückschicht (2) sowie eine unmittelbar vor Gebrauch ablösbare Schutzschicht (3) umfasst.

**13.** Vorrichtung zur transdermalen Verabreichung der freien Base von (R)- 2-[3-(1,1-Diisopropylamino)-1-phenylpropyl]-4-(hydroxymethyl)phenyl isobutyrat (Fesoterodin) über einen Zeitraum von mindestens 24 Stunden in einer konstanten Fluxrate von mindestens 4 $\mu$g/cm$^2$/Stunde.

**14.** Verwendung von (R)-2-[3-(1,1-Diisopropylamino)-1-phenylpropyl]-4-(hydroxymethyl)phenyl isobutyrat (Fesoterodin) zur Herstellung eines Arzneimittels zur transdermalen Verabreichung von Fesoterodin zur Vorbeugung oder Behandlung von Inkontinenz, insbesondere Dranginkontinenz, Hyperaktivität des Detrusors, Pollakisurie, Nykturie oder imperativem Harndrang, **dadurch gekennzeichnet, dass** Fesoterodin im Arzneimittel in einer Polymermatrix vorliegt.

**15.** Verwendung nach Anspruch 14, **dadurch gekennzeichnet, dass** Fesoterodin der Polymermatrix in Form der freien Base zugesetzt wird.

**16.** Verwendung nach einem der vorhergehenden Ansprüche, **dadurch gekennzeichnet dass** die Polymermatrix selbstklebend ist.

**17.** Verwendung nach einer der vorliegenden Ansprüche, **dadurch gekennzeichnet, dass** die wirkstoffhaltige Polymermatrix im Heißschmelzverfahren hergestellt wird.

**18.** Verwendung nach einer der vorliegenden Ansprüche, **dadurch gekennzeichnet, dass** die wirkstoffhaltige Polymermatrix im Lösemittelverfahren hergestellt wird.

**19.** Verwendung nach einem der vorhergehenden Ansprüche, **dadurch gekennzeichnet, dass** Fesoterodin in einer Dosis von mindestens 3 mg über mindestens 24 Stunden in konstanter Fluxrate durch Humanhaut freigesetzt wird.

**20.** Verwendung nach einem der vorhergehenden Ansprüche, wobei die Polymermatrix einen Haftkleber aus der Gruppe der Polyacrylate, Ethylenvinylactetate (EVA), Polyisobutylene, Silikone oder Styrolblock-Copolymere (SxS) umfasst.

**21.** Verwendung nach einem der vorhergehenden Ansprüche, **dadurch gekennzeichnet, dass** die Polymermatrix 50-95 Gew% eines Haftklebers enthält, der ausgewählt ist aus der Gruppe

(a) der Polyacrylate
(b) der EVA-Haftkleber,
(c) der Silikon-Haftkleber,
(d) der SXS-Haftkleber,
(e) der PIB-Haftkleber,

wobei den hydrophoben Haftklebern (c), (d) und (e) jeweils 2-20 Gew%, bezogen auf das Gesamtgewicht der Polymermatrix, eines hydrophilen Polymers zugesetzt sind.

**22.** Verwendung nach einem der vorhergehenden Ansprüche, wobei die Vorrichtung

(a) eine Grundfläche von maximal 50 cm$^2$ aufweist,

(b) eine selbstklebende Polymermatrix umfasst, die

(b1) ein Gewicht von 30-300 g/$m^2$ aufweist,
(b2) 50-95 Gew% eines Haftklebers enthält,
(b3) Fesoterodin in einer Konzentration von 5-40 Gew% bezogen auf das Gesamtgewicht der Polymermatrix enthält und

(c) Fesoterodin über einen Zeitraum von mindestens 24 Stunden bei einer konstanten Fluxrate von mindestens 4 μg/$cm^2$/h durch Humanhaut abgibt.

**23.** Herstellung einer Vorrichtung nach einem der Ansprüche 1-13, **dadurch gekennzeichnet, dass** Fesoterodin in Form der freien Base in eine Polymermatrix eingebracht wird.

## Claims

**1.** A device for the transdermal delivery of (R)-2-[3-(1,1-diisopropylamino)-1-phenylpropyl]-4-(hydroxymethyl)phenyl isobutyrate (Fesoterodine) **characterized by** the fact that Fesoterodine is present in a polymer matrix and is released through the human skin in a dose of 0.5-20 mg per day.

**2.** A device according to claim 1 **characterized by** the fact that the device is manufactured by Fesoterodine being added to the polymer matrix in the form of the free base.

**3.** A device according to one of the previous claims **characterized by** the fact that the polymer matrix incorporates 55-90 percent by weight of a contact adhesive and is self-adhesive.

**4.** A device according to one of the previous claims **characterized by** the fact that the polymer matrix incorporates one or several contact adhesives which are chosen from the group of acrylates, ethylene vinyl acetates (EVA), silicones or styrene block copolymers (SXS).

**5.** A device according to one of the previous claims **characterized by** the fact that the polymer matrix consists of up to 50-95 percent by weight of a hot-meltable mixture of a silicone based contact adhesive and at least one softener.

**6.** A device according to one of the previous claims **characterized by** the fact that the polymer matrix consists of up to 50-95 percent by weight from (a) a hydrophilic contact adhesive and/or (b) a mixture of a hydrophobic contact adhesive with 2-20 percent by weight, based on the total weight of the polymer matrix, of a hydrophilic polymer and/or (c) a mixture of a hydrophilic with a hydrophobic contact adhesive.

**7.** A device according to claim 6 whereby the hydrophilic polymer is PEO, PVP or PVAc.

**8.** A device according to one of the previous claims **characterized by** the fact that Fesoterodine was introduced into the polymer matrix in a degree of purity of above 97 percent by weight.

**9.** A device according to one of the previous claims **characterized by** the fact that the device

(a) exhibits a surface of a maximum 50 $cm^2$,
(b) comprises a self-adhesive polymer layer, which
(b) comprises a self-adhesive polymer layer, which

(b1) exhibits a weight of 30-300 g/$m^2$,
(b2) contains 50-95% by weight of a contact adhesive,
(b3) contains Fesoterodine in a concentration of 5-40 percent by weight based on the total weight of the polymer matrix and

(c) delivers Fesoterodine with a steady flux rate of at least 4 μg/$cm^2$/hour through the human skin over a time period of at least 24 hours.

**10.** A device according to one of the previous claims **characterized by** the fact that the device exhibits a base area of

a maximum of 40 $cm^2$, and the loading of the active ingredient of the self-adhesive polymer matrix amounts to 7-30 percent by weight.

**11.** A device according to one of the previous claims **characterized by** the fact that the device transports Fesoterodine in a dose of at least 3 mg per day over at least 24 hours at a constant flux rate through the human skin.

**12.** A device according to one of the previous claims **characterized by** the fact that the device incorporates an adhesive matrix containing an active ingredient (1), a backing (2) being impermeable and inert for the constituents of the adhesive matrix as well as a protective layer (3) detachable immediately before use.

**13.** A device for the transdermal delivery of the free base of (R)- 2-[3-(1,1-diisopropylamino)-1-phenylpropyl)-4-(hydroxymethyl)phenyl isobutyrate (Fesoterodine) over a time period of at least 24 hours at a constant flux rate of at least 4$\mu$g/$cm^2$/hour.

**14.** Use of (R)- 2-[3-(1,1-diisopropylamino)-1-phenylpropyl]-4-(hydroxymethyl)phenyl isobutyrate (Fesoterodine) for the manufacture of a medicine for the transdermal delivery of Fesoterodine for the prevention or treatment of incontinence, particularly urge incontinence, hyperactivity of the detrusor, pollakisuria, nocturia or imperative urinary urgency, **characterized by** the fact that Fesoterodine is present in the medicine in a polymer matrix.

**15.** Use according to claim 14, **characterized by** the fact that Fesoterodine is added to the polymer matrix in the form of the free base.

**16.** Use according to one of the previous claims **characterized by** the fact that the polymer matrix is self-adhesive.

**17.** Use according to one of the previous claims **characterized by** the fact that the polymer matrix containing the active ingredient is manufactured in a hot melt procedure.

**18.** Use according to one of the previous claims **characterized by** the fact that the polymer matrix containing the active ingredient is manufactured in a solvent procedure.

**19.** Use according to one of the previous claims **characterized by** the fact that Fesoterodine is released in a dose of at least 3 mg per day over at least 24 hours at a constant flux rate through human skin.

**20.** Use according to one of the previous claims, whereby the polymer matrix comprises a contact adhesive from the group of polyacrylates, ethylene vinyl acetates (EVA), polyisobutylenes, silicones or styrene block copolymers (SXS).

**21.** Use according to one of the previous claims **characterized by** the fact that the polymer matrix contains 50-95 percent by weight of a contact adhesive that is selected from the group

(a) of polyacrylates
(b) of EVA-contact adhesives,
(c) of silicone adhesives,
(d) of SXS-adhesives,
(e) of PIB-contact adhesives,

whereby 2-20 percent by weight of a hydrophilic polymer is added to each of the hydrophobic contact adhesives (c), (d) and (e) based on the total weight of the polymer matrix.

**22.** Use according to one of the previous claims, whereby the device

(a) exhibits a surface of a maximum 50 $cm^2$,
(b) comprises a self-adhesive polymer matrix, which

(b1) exhibits a weight of 30-300 $g/m^2$,
(b2) contains 50-95% by weight of a contact adhesive,
(b3) contains Fesoterodine in a concentration of 5-40 percent by weight based on the total weight of the polymer matrix and

(c) delivers Fesoterodine with a steady flux rate of at least 4 μg/cm$^2$/hour through the human skin over a time period of at least 24 hours.

**23.** Manufacture of a device according to one of claims 1-13, **characterized by** the fact that Fesoterodine is introduced into a polymer matrix in the form of the free base.

**Revendications**

**1.** Dispositif pour l'administration transdermique d'isobutyrate de (R)-2-[3-(1,1-di-isopropylamino)-1-phénylpropyl]-4-(hydroxyméthyl)-phényle (Fésotérodine), ***caractérisé en ce que*** la Fésotérodine est présente dans une matrice de polymère et est libérée selon une dose de 0,5 à 20 mg par jour à travers la peau humaine.

**2.** Dispositif selon la revendication 1, ***caractérisé en ce que*** le dispositif est fabriqué en ajoutant la Fésotérodine à la matrice de polymère sous la forme de la base libre.

**3.** Dispositif selon l'une quelconque des revendications précédentes, ***caractérisé en ce que*** la matrice de polymère contient de 50 à 95 % en poids d'un adhésif et est autocollante.

**4.** Dispositif selon l'une quelconque des revendications précédentes, ***caractérisé en ce que*** la matrice de polymère contient un ou plusieurs adhésifs choisi dans le groupe composé des acrylates, des copolymères éthylène-acétate de vinyle (EVA), des silicones ou des copolymères-blocs de styrène (SXS).

**5.** Dispositif selon l'une quelconque des revendications précédentes, ***caractérisé en ce que*** la matrice de polymère se compose d'entre 50 et 95 % en poids d'un mélange thermofusible d'un adhésif silicone et d'au moins un plastifiant.

**6.** Dispositif selon l'une quelconque des revendications précédentes, ***caractérisé en ce que*** la matrice de polymère se compose entre 50 et 95 % en poids (a) d'un adhésif hydrophile et/ou (b) d'un mélange d'un adhésif hydrophobe avec 2-20 % en poids, par rapport au poids total de la matrice de polymère, d'un polymère hydrophile, et/ou (c) d'un mélange d'un adhésif hydrophile et d'un adhésif hydrophobe.

**7.** Dispositif selon la revendication 6, dans lequel le polymère hydrophile est du PEO, du PVP ou du PVAc.

**8.** Dispositif selon l'une quelconque des revendications précédentes, ***caractérisé en ce que*** la Fésotérodine est intégrée dans la matrice de polymère avec une pureté de plus de 97 %.

**9.** Dispositif selon l'une des revendications précédentes, ***caractérisé en ce que*** le dispositif :

(a) a une surface de 50 cm$^2$ au maximum,
(b) contient une couche de polymère autocollante, qui

(b1) a un poids de 30 à 300 g/m$^2$,
(b2) contient de 50 à 95 % en poids d'un adhésif,
(b3) contient de la Fésotérodine à une concentration de 5 à 40 % en poids par rapport au poids total de la matrice de polymère et

(c) libère la Fésotérodine sur une durée d'au moins 24 heures à un flux constant d'au moins 4 μg/cm$^2$/heure à travers la peau humaine.

**10.** Dispositif selon l'une quelconque des revendications précédentes, ***caractérisé en ce que*** le dispositif a une surface de 40 cm$^2$ au maximum et la charge de principe actif de la matrice de polymère autocollante est de 7 à 30 % en poids.

**11.** Dispositif selon l'une quelconque des revendications précédentes, ***caractérisé en ce que*** le dispositif fait passer un composé selon la formule I à travers la peau humaine selon une dose d'au moins 3 mg par jour pendant au moins 24 heures, à un flux constant.

**12.** Dispositif selon l'une quelconque des revendications précédentes, ***caractérisé en ce que*** le dispositif comprend une matrice adhésive (1) contenant le principe actif, une couche de support (2) inerte et imperméable aux composants

de la matrice adhésive et une couche de protection (3) pouvant être détachée immédiatement avant usage.

**13.** Dispositif pour l'administration transdermique de la base libre d'isobutyrate de (R)-2-[3-(1,1-di-isopropylamino)-1-phénylpropyl]-4-(hydroxyméthyl)-phényle (Fésotérodine) pendant au moins 24 heures à un flux constant d'au moins 4 $\mu g/cm^2$/heure.

**14.** Utilisation de l'isobutyrate de (R)-2-[3-(1,1-di-isopropylamino)-1-phénylpropyl]-4-(hydroxyméthyl)-phényle (Fésotérodine) pour la fabrication d'un médicament pour l'administration transdermique de Fésotérodine en vue de la prévention ou du traitement de l'incontinence, en particulier de l'incontinence d'effort, de l'hyperactivité du détrusor, de la pollakiurie, de la nycturie ou des besoins impérieux d'uriner, ***caractérisée en ce que*** la Fésotérodine est contenue dans le médicament dans une matrice de polymère.

**15.** Utilisation selon la revendication 14, ***caractérisée en ce que*** la Fésotérodine est ajoutée à la matrice de polymère sous la forme de la base libre.

**16.** Utilisation selon l'une quelconque des revendications précédentes, ***caractérisée en ce que*** la matrice de polymère est autocollante.

**17.** Utilisation selon l'une quelconque des revendications précédentes, ***caractérisée en ce que*** la matrice de polymère contenant le principe actif est fabriquée selon le procédé de fusion à chaud.

**18.** Utilisation selon l'une quelconque des revendications précédentes, ***caractérisée en ce que*** la matrice de polymère contenant le principe actif est fabriquée selon le procédé avec solvants.

**19.** Utilisation selon l'une quelconque des revendications précédentes, ***caractérisée en ce que*** la Fésotérodine est libérée à travers la peau humaine selon une dose d'au moins 3 mg pendant au moins 24 heures, à un flux constant.

**20.** Utilisation selon l'une quelconque des revendications précédentes, dans laquelle la matrice de polymère contient un adhésif choisi dans le groupe composé des polyacrylates, des copolymères éthylène-acétate de vinyle (EVA), du polyisobutylène, des silicones ou des copolymères-blocs de styrène (SxS).

**21.** Utilisation selon l'une quelconque des revendications précédentes, ***caractérisée en ce que*** la matrice de polymère contient de 50 à 95 % en poids d'un adhésif choisi dans le groupe comprenant

(a) les polyacrylates,
(b) les adhésifs EVA,
(c) les adhésifs silicones,
(d) les adhésifs SXS,
(e) les adhésifs PIB,

les adhésifs hydrophobes (c), (d) et (e) étant additionnés de 2 à 20 % en poids, par rapport au poids total de la matrice de polymère, d'un polymère hydrophile.

**22.** Utilisation selon l'une quelconque des revendications précédentes, dans laquelle le dispositif

(a) a une surface de 50 $cm^2$ au maximum,
(b) contient une couche de polymère autocollante, qui

(b1) a un poids de 30 à 300 $g/m^2$,
(b2) contient de 50 à 95 % en poids d'un adhésif,
(b3) contient de la Fésotérodine à une concentration de 5 à 40 % en poids par rapport au poids total de la matrice de polymère et

(c) libère la Fésotérodine sur une durée d'au moins 24 heures à un flux constant d'au moins 4 $\mu g/cm^2$/heure à travers la peau humaine.

**23.** Fabrication d'un dispositif selon l'une quelconque des revendications 1 à 13, ***caractérisée en ce que*** la Fésotérodine est ajoutée à la matrice de polymère sous la forme de la base libre.

Abbildung 1/4

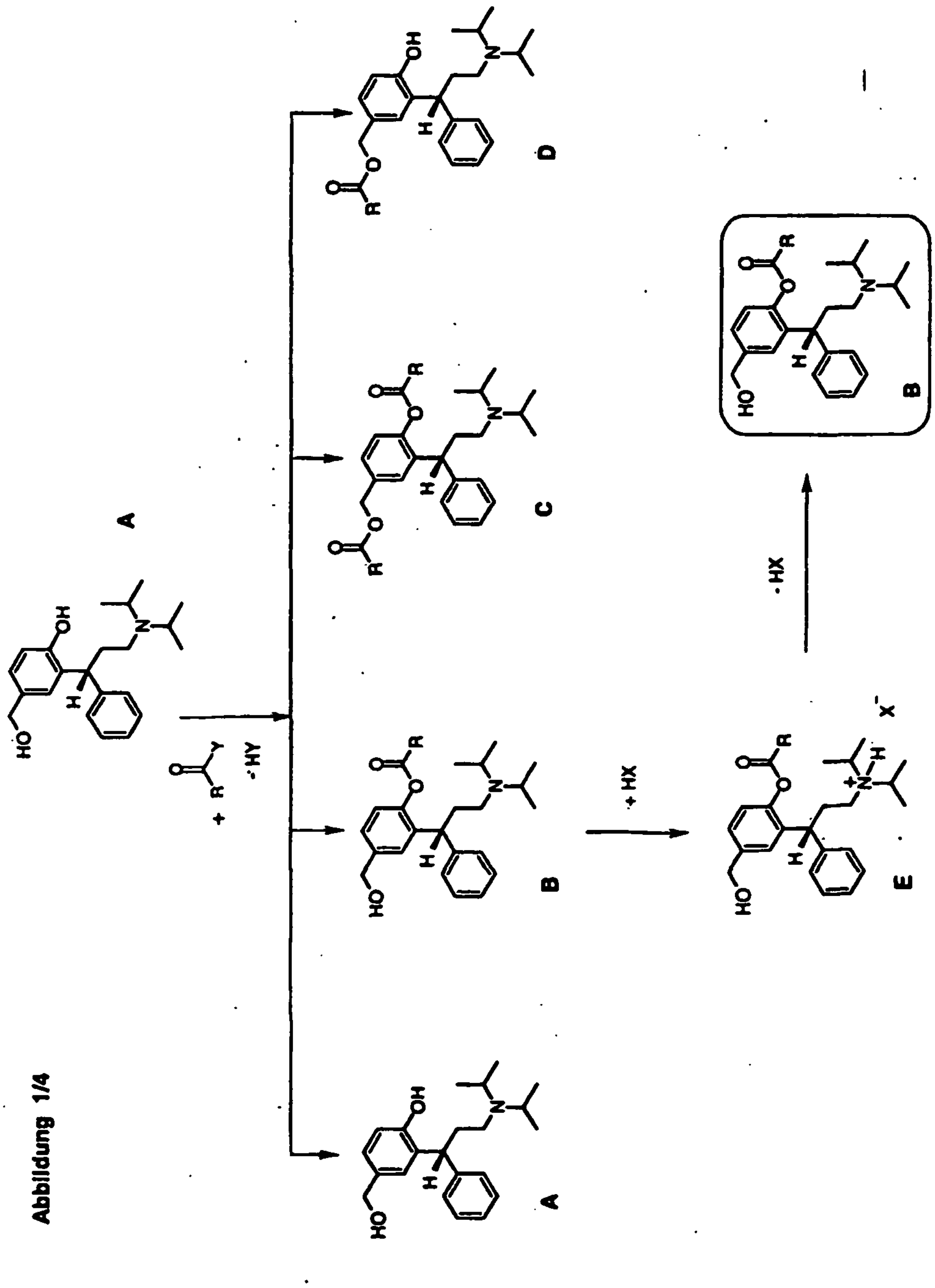

## Abbildung 2/4:

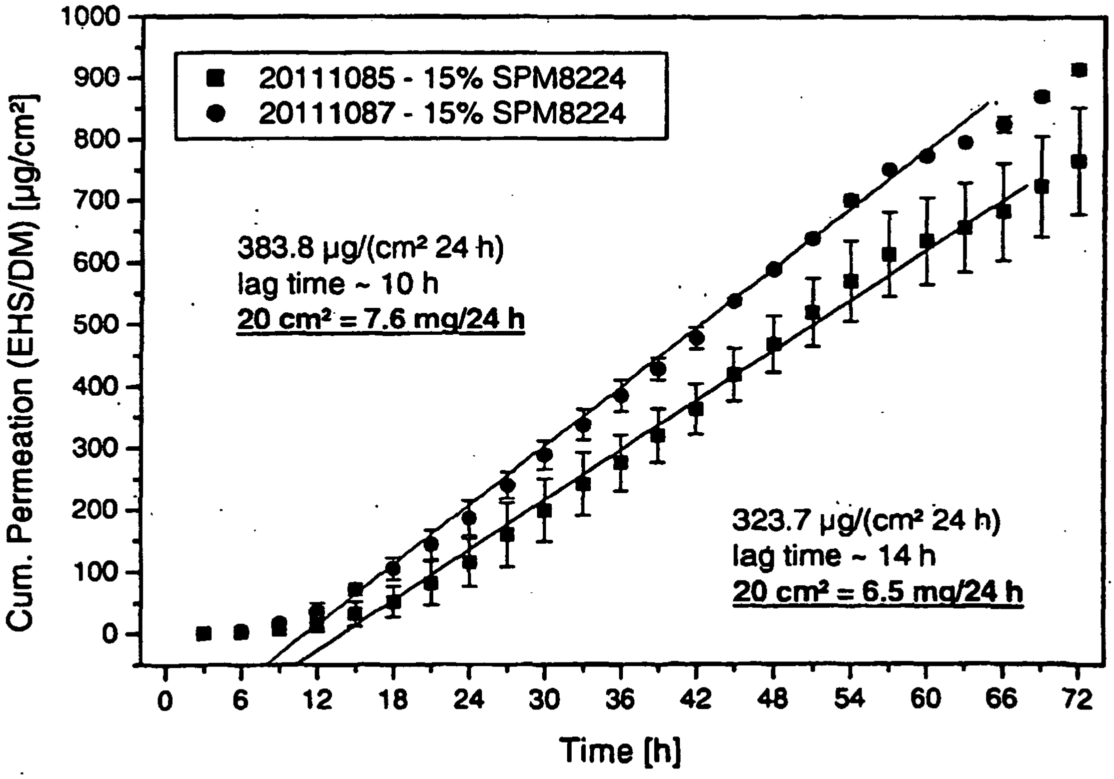

## Abbildung 3/4

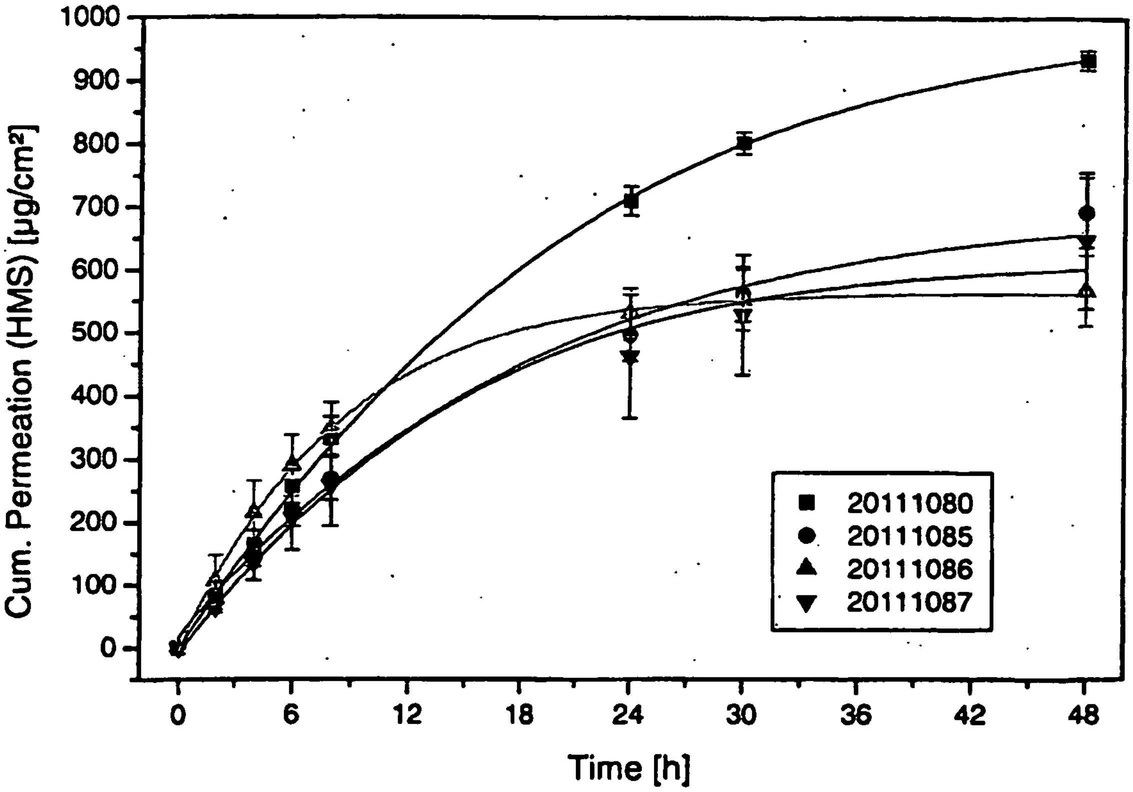

**Abbildung 4/4**

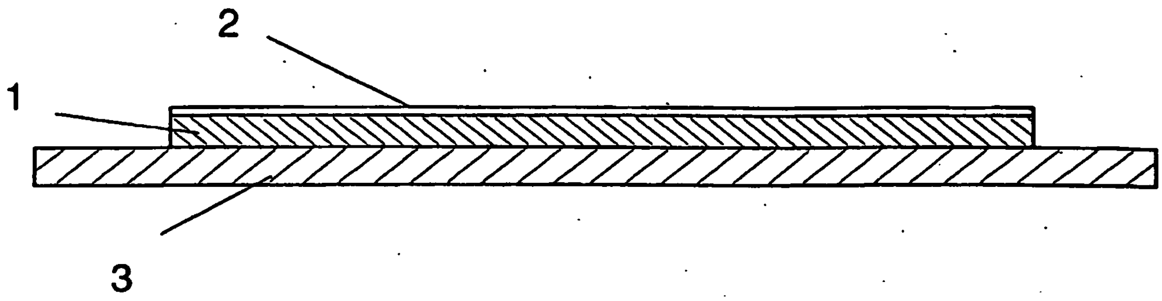


Schematischer Aufbau eines monolithischen TTS

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9958478 A
- WO 0135957 A
- WO 9407486 A
- US 5328696 A
- EP 180377 A
- US RE35474 E
- WO 9949852 A
- WO 9948493 A
- EP 835136 A
- EP 360467 A
- EP 524775 A
- EP 663431 A
- US RE36754 E
- US 4144317 A
- US 5559165 A
- US 5527536 A
- US 5498418 A


### In der Beschreibung aufgeführte Nicht-Patentliteratur


- **COLUCCI.** *Annals of Pharmacotherapy,* 1999, vol. 33, 1173
- **BRYNNE, BR.** *J Clin Pharmacol,* 1999, vol. 48, 564
- *Acta Pharm Technol,* 1999, vol. 34, 99
- Parameters for Compartment-free Pharmacokinetics. Shaker Verlag, 1999, 112
- **H. TANOJO et al.** *J. Control Rel.,* 1997, vol. 45, 41-47